(19) Europäisches Patentamt / European Patent Office / Office européen des brevets

(11) **EP 2 333 502 B1**

(12) **EUROPEAN PATENT SPECIFICATION**

(45) Date of publication and mention
of the grant of the patent:
**13.08.2014   Bulletin 2014/33**

(21) Application number: **10194413.0**

(22) Date of filing: **09.12.2010**

(51) Int Cl.:
*G01J 5/00* (2006.01)          *G01N 25/72* (2006.01)
*C03B 9/41* (2006.01)          *G01J 5/02* (2006.01)
*G01J 5/08* (2006.01)          *G01J 5/10* (2006.01)
*G01N 33/38* (2006.01)

(54) **System and method for monitoring hot glass containers to enhance their quality and control the forming process**

System und Verfahren zum Überwachen von heißen Glasbehältern zur Verbesserung ihrer Qualität und Steuerung des Formverfahrens

Système et procédé de commande de conteneurs de verre chaud pour améliorer leur qualité et contrôler le processus de formation

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **10.12.2009   EP 09075545**

(43) Date of publication of application:
**15.06.2011   Bulletin 2011/24**

(73) Proprietor: **Emhart Glass S.A.
6330 Cham (CH)**

(72) Inventors:
• **Holtkamp, Mark Edwin
  9721 AM Groningen (NL)**
• **Brummelman, Teunis Rene
  9617 EC Harkstede (NL)**

(74) Representative: **Johnstone, Douglas Ian
  Baron Warren Redfern
  Cambridge House
  100 Cambridge Grove
  Hammersmith
  London
  W6 0LE (GB)**

(56) References cited:
EP-A- 0 643 297          EP-A1- 1 494 012
WO-A1-00/56673          WO-A1-2004/011935
GB-A- 2 149 910          JP-A- 2003 083 718
US-A- 3 968 368          US-A- 4 064 534
US-A- 5 345 389          US-A- 5 583 337
US-A1- 2004 262 523          US-A1- 2007 102 628
US-B1- 6 212 962          US-B1- 6 584 805
US-B2- 7 054 710

• DR JOHN CHAN: "Automated inspection and
container monitoring at the hot end",
INTERNATIONAL GLASS REVIEW, CONTRACT
COMMUNICATIONS, LONDON, GB, 1 January
1997 (1997-01-01), pages 109-111, XP002991197,
ISSN: 1359-4974

EP 2 333 502 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

Field of the Invention

The present invention relates generally to a system and method for monitoring hot glass containers at the hot end as they stream from an I.S. (Individual Section) machine manufacturing them to enable their quality to be monitored, enhanced, and controlled.

Glass containers are made in a manufacturing process that has three parts, namely the batch house, the hot end, and the cold end. The batch house is where the raw materials for glass (typically including sand, soda ash, limestone, cullet (crushed, recycled glass), and other raw materials) are prepared and mixed into batches. The hot end begins with a furnace, in which the batched materials are melted into molten glass, and from which a stream of molten glass flows.

The molten glass is cut into cylinders of glass called gobs, which fall by gravity into blank molds. In the blank molds, a pre-container referred to as a parison is formed, either by using a metal plunger to push the glass into the blank mold, or by blowing the glass from below into the blank mold. The parison is inverted and transferred to a mold, where the parison is blown out into the shape of the container. The hot end also includes an annealing process which prevents the containers from having weakened glass caused by stresses caused by uneven cooling. The annealing process is used to achieve even cooling, using an annealing oven or Lehr to heat the containers, and then slowly cool them over a twenty to sixty minute period.

The equipment at the cold end of the glass container manufacturing process inspects the containers to ensure that they are of acceptable quality. All glass containers are inspected by automated machines after manufacturing for a variety of faults. Those skilled in the art will appreciate that if there is a problem in the way that glass containers are being molded by the I.S. machine, unless that problem is readily apparent from a casual inspection of the glass containers as they stream from the I.S. machine on conveyors that take them from the I.S. machine, through the annealing oven or Lehr, and through the inspection equipment at the cold end, there may be thousands of defective glass containers that require scrapping by the time that the existence of a problem is first determined.

Accordingly, it is beneficial to determine the existence of a recurring quality issue in glass containers as close as possible to the time that they exit the I.S. machine and while they are still very hot. Several attempts to inspect hot glass containers at the hot end of the glass container manufacturing line have been made. An early attempt is illustrated in U.S. Patent No. 5,583,337, granted on December 10, 1996, to Chan, which used a camera sensitive to infrared radiation located opposite the travel of the hot glass container through an inspection zone. The image of each hot glass container was processed into regions and data for each region was compared to predetermined parameters in order to determine whether or not the hot glass container was defective.

Another early attempt is illustrated in European Patent No. 643,297, granted on December 18, 2002, to Troost, which used one or two infrared cameras and compared infrared energy distribution and/or energy differences in images from the infrared camera with a mathematical reference model that had been developed by means of specific physical properties, such as the released (emitted) infrared radiation in combination with specific sizes and glass composition of the product.

Another attempt is illustrated in U.S. Patent No. 6,188,079, granted on February 13, 2001, to Juvinall et al., which measured the intensity of radiation emitted by hot glass containers at two different wavelengths, namely a near-infrared wavelength where the image varied as a function of both surface temperature and wall thickness, and a higher wavelength infrared wavelength where the image varied as a function of surface temperature only. Wall thickness was determined by comparing the images with each other.

A more recent attempt is illustrated in U.S. Patent Application Publication No. 2006/0096319, published on May 11, 2006, to Dalstra, which uses near-infrared wavelength radiation from hot glass containers to obtain an image of the hot glass containers. Each image is subdivided into at least two regions, average intensity values for the regions for each hot glass container image are determined, and the average intensity value of each region for each hot glass container is compared with a reference value such as "the running average" to determine deviations therefrom, from which an error signal may be generated additionally, a cooling curve is calculated and used as a reference to compensate for the difference in the amount of radiation of glass products due to different cooling times.

All of these systems may generate error signals even when there is a change in the amount of infrared radiation that is not brought about due to a change in the forming process, but is due instead to changes in environmental and other conditions and parameters, such as, for example, the ambient temperature, the ambient humidity, the cooling air temperature, the cooling air humidity, smoke and dirt in the air, the infrared camera settings, contamination of the infrared camera optics, the production speed, the glass material composition, and glass container weight. These conditions and parameters, which as such have nothing to do with glass container quality, can drastically alter the measured infrared radiation intensities depending on, for example, whether the system is operating at day or night, differences occasioned by different seasons, the production location, and/or the I.S. machine itself.

Consequently, the operator must always be present to monitor the measurement results and the generated error signals carefully, to check the conditions and parameters, and to adjust reference values in order to compensate for continuously changing conditions and parameters. From a practical standpoint, this is highly undesirable, since labor costs are relatively high and the glass container manufacturing process occurs in a hot and noisy environment, which is not a favorable labor environment.

Another disadvantage of the known systems, and particular the Troost and Dalstra systems, is that when starting up the production of a glass container that has been produced earlier, the above mentioned conditions and parameters may have been changed, in which case the reference values and/or cooling curves used for the previous production may not be useful for the current production (and, if used, may actually produce glass containers that are of an unacceptable quality). For these systems, each time a new master reference and/or a new cooling curve is required, the startup time will be lengthened considerably, which is not desirable.

The inventors of the present invention in their previous European Patent Application No. EP 09075545.5, filed on December 10, 2009, published as EP 2336740A and assigned to the assignee of the present patent application, presented a substantial improvement to the systems that were mentioned above. That invention obtained infrared images of hot glass containers having a predetermined number of horizontal scan image lines with each pixel on each image line having a digital value. A value for the line radiation measurement for each image line was determined by summing the digital values of all of the pixels in each image line. A total radiation measurement for each hot glass container was obtained by summing the digital values of all of the pixels in all of the image lines for the hot glass container. By dividing each of the line radiation measurements by the total radiation measurement, the values for each glass container were normalized without the necessity of comparing them to an average value, a master reference value, or a cooling curve.

While this invention represents a tremendous advancement over the other systems that were mentioned above, further enhancements that have been made will be described hereinbelow. These further advancements will be directed to presenting images to an operator on a touchscreen user interface module that will enable the quick identification of glass forming process deviations including the geometry of the glass containers and the distribution of glass in the glass containers, provide forming process quality feedback, identify instant and formerly "unseen" potential improvements, provide early and adequate warnings of quality issues, and to occasion continuous improvements in glass container quality.

The present invention is directed to a method and system for monitoring and analysing characteristics of hot glass containers formed by an I.S. machine, generally of the type disclosed in aforementioned US-A-5,583,337, and as defined in the preambles of claims 1 and 22 respectively.

According to the present invention there are provided a method and system as defined in the characterising clauses of claims 1 and 22 respectively.

## SUMMARY OF THE INVENTION

The disadvantages and limitations of the background art discussed above are overcome by the present invention. With this invention, a system and a method are provided for monitoring a glass container forming process at the hot end that is independent of the mentioned conditions and parameters that have hampered previously known attempts to monitor hot glass containers and with which it is possible to produce glass containers of both a high quality and a substantially increased level of consistency. The system monitors radiation from the hot glass containers, extracts images of each hot glass container, analyzes the images of the hot glass containers, and provides the images of the hot glass containers together with information indicative of the quality thereof to a display screen viewable by an operator.

One or more Short Wave Infrared ("SWIR") imaging device are positioned directly after the I.S. machine on opposing sides of the container to monitor hot glass containers as they are formed by the I.S. machine and pass by the SWIR imaging device(s) on the conveyor. The SWIR imaging device(s) provide electronic images from which an electronic image for each hot glass container is extracted. The electronic images of the hot glass containers are then processed to identify cavities that stand out from the overall population of all cavities using a number of different criteria that are relevant to the quality of the hot glass containers. In a preferred embodiment, at least some of the information coming from the hot glass containers may be normalized using the techniques taught in EP 2336740A referred to above. This normalizes the measurement from hot glass container to hot glass container and thereby removes the effects of overall temperature variations between glass containers, changing ambient conditions, and other variations affecting the measurements, which provides a unique quality reference for each glass container.

The processing performed on the images of the hot glass containers provides a wide variety of information regarding the hot glass containers to quickly identify deviations in the glass forming process and glass distribution throughout the hot glass containers. The images produced by the SWIR imaging devices are processed to identify cavities that stand out from the overall population of all cavities. The deviations used to identify the outliers are based upon the containers vertical and horizontal glass distribution, dimensional outline including lean, and the position on the conveyor. Cavities or sections producing outlying containers are quickly identified and visually displayed to the machine operator.

Some of this information is stored by the hot glass container quality analytical system in databases. Both the images of the hot glass containers and the analyses of the hot glass containers are presented on a user interface screen. In the preferred embodiment, the user interface screen is a touchscreen that allows a user to interact with the images and obtain analytical information presented with the images.

One of the features provided on the user interface screen is live views of the hot glass containers that are updated in real time. In a preferred embodiment, all the hot glass containers from all of the molds in all of the sections of the I.S. machine may be viewed simultaneously in real time. The hot glass container quality analytical system also displays annotations indicative of characteristics that are problematic, including displaying alarms and indications that containers are being rejected by the system. In one embodiment, the worst molds may be displayed to the user of the system to allow diagnosis and correction of the performance issues.

The Screens also include displays of vertical glass distribution and horizontal glass distribution, and in one preferred embodiment both vertical glass distribution and horizontal glass distribution are displayed simultaneously in a single display. This display is also presented in real time. In another preferred embodiment, the analytical information derived from the hot glass containers may be provided to the I.S. machine to enable automatic correction of some aspects of the glass container manufacturing process, including placement of the glass containers on the conveyor.

The hot glass container quality analytical system of the present invention thus presents images together with analytical information regarding the quality of the hot glass containers to an operator on a touchscreen display that enables the quick identification of glass forming process deviations including the geometry of the glass containers, the distribution of glass in the glass containers, and the positions of the glass containers on the conveyor. Forming process quality feedback is also provided, thereby identifying instant and formerly "unseen" potential improvements, providing early and adequate warnings of quality issues, and contributing to continuous improvements in glass container quality.

## DESCRIPTION OF THE DRAWINGS

These and other advantages of the present invention are best understood with reference to the drawings, in which:

FIG. 1 is a schematic view of a forming machine and an embodiment of the analytical system;

FIG. 2 is a an image of a glass container;

FIG. 3 is a line radiation measurement for the glass container shown in FIG. 1;

FIG. 4 is a measurement-ratio curve for the glass container shown in FIG. 2;

FIG. 5 is a reference curve for the glass container shown in FIG. 2;

FIG. 6 is the reference curve together with the measurement-ratio curve for the glass container shown in FIG. 2;

FIG. 7 is a relative difference curve for the glass container shown in FIG. 2;

FIG. 8 is a simplified view of a hot glass container quality analytical system according to the present invention showing the essential components thereof as installed in a typical glass container manufacturing line;

FIG. 9 is a schematic view showing the operational connections of the essential components of the exemplary hot glass container quality analytical system illustrated in FIG. 8 showing the flow of data between the components;

FIG. 10 is a schematic depiction illustrating the operation of one of the camera modules illustrated in FIG. 9 at a high level;

FIG. 11 is a schematic depiction showing the operation of the first camera module illustrated in FIG. 9 at a more detailed level than the level illustrated in FIG. 10;

FIG. 12 is a functional schematic depiction of the image processing module illustrated in FIG. 11;

FIG. 13 is a schematic drawing of a plurality of hot glass containers in a continuing digital "filmstrip" of images showing an image of a single hot glass container to be extracted from the "filmstrip" of images by the image processing module functionally illustrated in FIG. 12;

FIG. 14 is a is a schematic drawing of a hot glass container extracted from the image of a single hot glass container in the "filmstrip" of images illustrated in FIG. 13;

FIG. 15 is a side view of a "stuck ware" in which two still-plastic glass containers have become stuck together;

FIG. 16 is a side view of a "down ware" in which a glass container has fallen down on the conveyor;

FIG. 17 is a schematic top plan view of a portion of the conveyor showing longitudinal and lateral locations of each of a plurality of glass containers in a corresponding plurality of longitudinal locations;

FIG. 18 is a schematic drawing of the border detection occurring to the image of the single hot glass container image extracted from the "filmstrip" of images in FIG. 14;

FIG. 19 is a side view of glass container showing a line extending through a determined middle of the glass container;

FIG. 20 is a side view of a glass container that is a "leaner," with the determination of lean for the glass container being illustrated by lines illustrating lean for the top and bottom halves of the glass container;

FIG. 21 is a side view of another glass container that is a "leaner," with a single line illustrating lean for the glass container;

FIG. 22 is a schematic depiction showing the formatted image data flow occurring in the image processing module illustrated in FIGS. 10 and 12;

FIG. 23 is a schematic depiction showing the combination of data occurring in the combine module illustrated in FIG. 12;

FIG. 24 is a schematic depiction showing the combination of object data that will be provided to the user interface modules and displayed thereupon;

FIG. 25 is a screenshot of the display of a touchscreen user interface module showing an Overview screen in which the last glass container imaged from each mold in each section of the I.S. machine is simultaneously displayed and updated in real time;

FIG. 26 is a screenshot of the Help screen for the Overview screen of FIG. 25, showing an index for many of the symbols used in the Overview screen;

FIGS. 27A-C are displays of a Vertical Distribution, a Horizontal Distribution, and a Glass Distribution with both Vertical Distribution and Horizontal Distribution, respectively;

FIG. 28 is a screenshot of the Glass Distribution screen which shows both Vertical Distribution and Horizontal Distribution;

FIG. 29 is a screenshot of the Help screen for the Glass Distribution screen of FIG. 28, providing explanations for the information that is displayed in the Glass Distribution screen;

FIG. 30 is a screenshot of the Attention screen which shows the three worst producing molds of the I.S. machine;

FIG. 31 is a screenshot of the Transport screen showing the real time location of hot glass containers on the conveyor for each mold as well as the variation in positions over a selected time period;

FIG. 32 is a screenshot of the Help screen for the Transport screen of FIG. 31, providing explanations for the information that is displayed in the Transport screen;

FIG. 33 is a screenshot of the Rejects screen showing summary information about the glass containers that have been rejected including the basis for their rejection;

FIG. 34 is a screenshot of the a Section Summary screen for one of the sections of the I.S. machine, displaying a large amount of information regarding glass containers produced by that section;

FIG. 35 is a screenshot of the Help for the Section Summary screen of FIG. 34, providing explanations for the information that is displayed in the Section Summary screen;

FIG. 36 is a screenshot of the Machine Variation Vertical Distribution screen showing summary information about the vertical distribution of glass in the glass containers produced by the I.S. machine;

FIG. 37 is a screenshot of the Machine Variation Horizontal Distribution screen showing summary information about the horizontal distribution of glass in the glass containers produced by the I.S. machine;

FIG. 38 is a screenshot of the Machine Variation Temperature screen showing summary information about the temperature of glass in the glass containers produced by the I.S. machine;

FIG. 39 is a screenshot of the Machine Variation Leaning screen showing summary information about the leaning of glass in the glass containers produced by the I.S. machine; and

FIG. 40 is a screenshot of the Statistics screen showing summary graphical information regarding the operation of the I.S. machine.

DETAILED DESCRIPTION OF THE EXEMPLARY EMBODIMENT

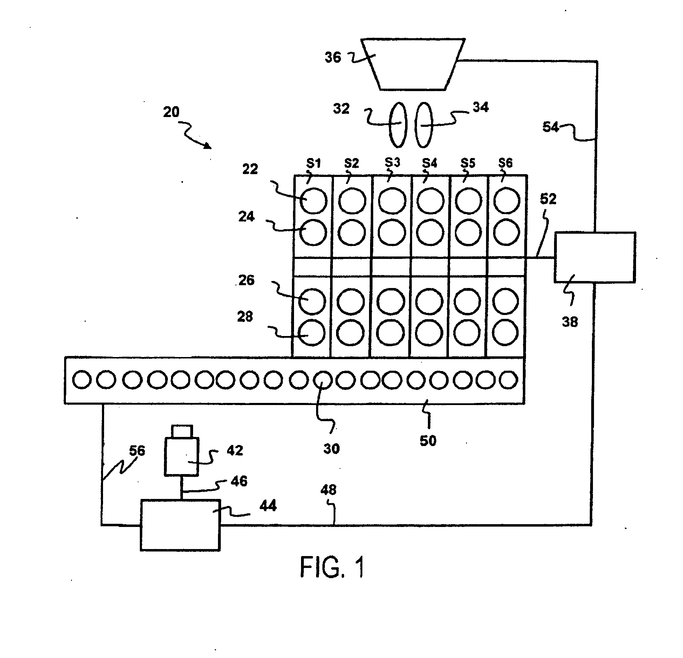

FIG. 1 shows an embodiment of the system where the glass container forming machine 20 contains six independent sections S1, S2, ... S6, each of which contains two forming stations 22 and 24. In one production cycle, the forming machine 20 produces twelve glass containers 30. Two molten glass gobs 32 and 34 are formed at the same moment by the feeder unit 36 and are loaded into the so-called blank moulds 22 and 24. Each section S1, S2, ... S6 of the forming machine 20 in this embodiment contains two blank molds 22 and 24 in which pre-containers or parisons are formed by pressing or blowing depending on the process type (press-blow or blow-blow). The formed parisons are transferred to the so-called blow molds 26 and 28 where the parisons are blown into the final shape of the glass containers 30. The mechanisms of the forming machine 20 and the feeder unit 36 are controlled by the control unit 38 through lines 52 and 54, respectively.

The glass containers 30 are transported by a conveyor belt 50 through a measurement unit 42 which takes images of the hot glass containers 30 and sends these images to a processor unit 44 through a line 46. Although in this embodiment one measurement unit 42 is used, the number of measurement units 42 may be increased depending on the circumstances and the accuracy to be achieved. However, even with one measurement unit, the achieved accuracy is fairly high.

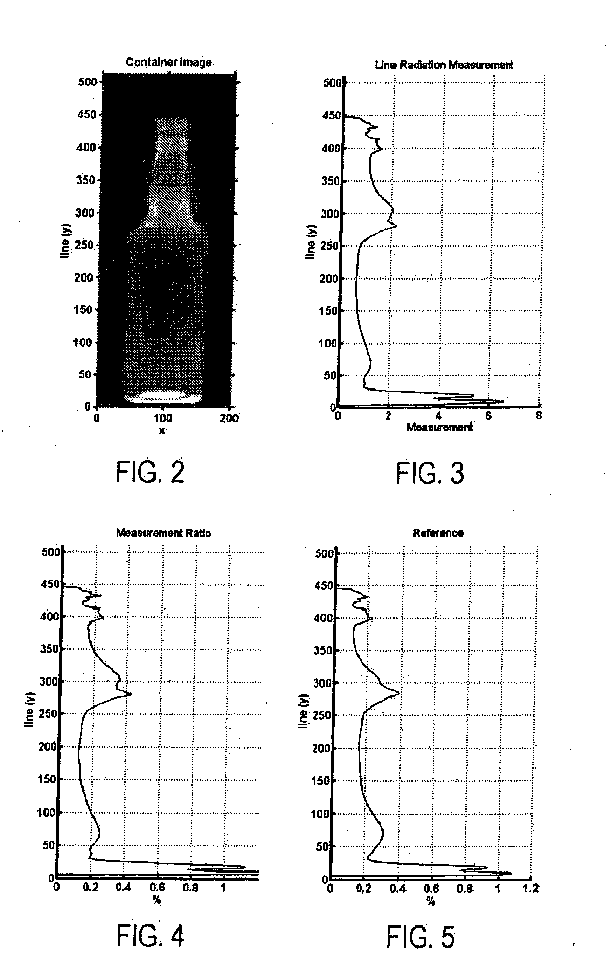

The measurement unit 42, an area camera in this embodiment, is preferably sensitive to Short Wave Infrared ("SWIR") radiation. Since radiation at wavelengths smaller than 3.0 microns from container glass is indicative of both the glass temperature and the glass thickness, a more accurate measurement may be obtained at wavelengths smaller than 3.0 microns, especially when analyzing relatively thicker glass containers. Therefore, a preferred embodiment of analytical system according to the present invention is that the measurement unit is sensitive to wavelengths of between 0.7 and 3.0 microns. More specifically, the measurement unit uses a Short Wave Infrared ("SWIR") camera, for example a 512 or 1024 pixels line-scan or area SWIR camera. The image taken by the camera of the hot glass container 30 shown in FIG. 2 may, for example, contain 512 image-lines, with each image-line for example containing 200 pixels.

The processor unit 44 determines for each glass container 30 the total radiation measurement by summing the digital values of all the pixels in the glass container image. The total radiation measurement of the glass container shown in FIG. 2 has a value of 553. Next, the processor unit 44 determines the line radiation measurements by summing for each image-line the digital values of all 200 pixels. The line radiation measurements belonging to the glass container image of FIG. 2 are shown in FIG. 3. Next, the processor unit 44, determines the measurement-ratio curve by dividing the line radiation measurements by the total radiation measurement, as shown hereunder:

$$I_{tot,s} = \sum I_{x,y,s} \quad (x = 1, 2, \dots 200, \; y = 1, 2, \dots, 512)$$

$$I_{y,s} = \sum I_{x,y,s} \quad (x = 1, 2, \dots 200)$$

$$I_{ratio,y,s} = (I_{y,s} / I_{tot,s}) * 100\%$$

Where:

$I_{tot,s}$ = the total radiation measurement value of a glass container image, originating from station s;

$I_{x,y,s}$ = the digital value of pixel x, y of the glass container image, originating from station s with y representing an image-line containing 200 x pixels, x=1 ... 200, y=1 ... 512, s=1 ... 12;

$I_{y,s}$ = the line radiation measurement value for image-line (y) of a glass container image, originating from station s; and

$I_{ratio,y,s}$ = the measurement-ratio value for image-line y of a glass container image, originating from station s.

The measurement-ratio values are expressed in percentages for clarity. The measurement-ratio curve depicted in FIG. 4 belongs to the glass container shown in FIG. 2. The order in which these steps occur can be varied as long as the same results are achieved. One can easily see that, for example, an attenuation $\alpha$ of the radiation received from the glass container 30 caused by an ambient parameter (for example smoke in the air) has no influence on the measurement-ratio curve:

$$I_{ration,y,s} = (\alpha I_{y,s} / \alpha I_{tot,s}) * 100\% = (I_{y,s} / I_{tot,s}) * 100\%$$

Next, the processor unit 44 determines a reference curve by determining the median values of measurement-ratio curves from a number of glass containers 30 from all or certain selected forming stations. This reference curve is unique for the glass container type produced.

The values of the reference curve are derived as illustrated below:

$$I_{reference,y} = (\sum_{k=1}^{N} I_{ratio,y,k}) / N$$

Where:

$I_{reference,y}$ = the reference curve value for line (y) ; and

$N$ = the number of glass containers 30 taken into account.

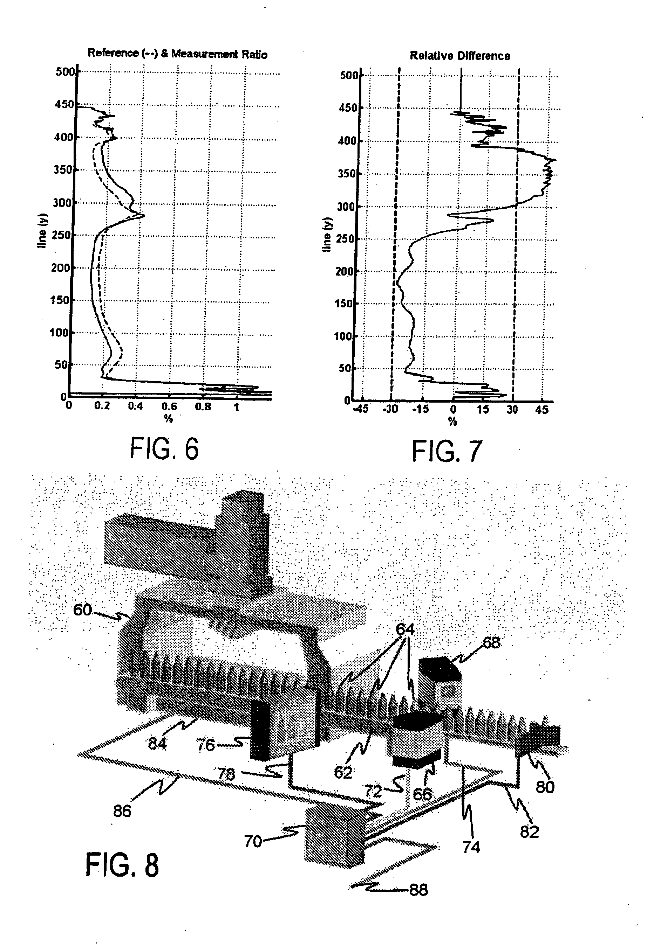

The reference curve may be stored and used later to decrease the time necessary to start up the production of the particular glass container 30 on the same or on another forming machine. The reference curve belonging to the glass container type in this example is shown in FIG. 5. In FIG. 6, the reference curve is shown together with the measurement-ratio curve of FIG. 4.

The processor unit 44 next determines the relative difference curve by subtracting the reference curve from the measurement-ratio curve and dividing the difference by the reference curve. This is illustrated hereunder:

$$\Delta I_{s,y} = ((I_{ratio,s} - I_{reference,y}) / I_{reference,y}) * 100\%$$

Where:

$\Delta I_{s,y}$ = the relative difference value at line y of a glass container image originating from the station s.

The relative difference curve shows how much and where the measurement-ratio curve of a glass container deviates from the reference curve. The processor unit 44 may display on a connected monitor (not shown) for each forming station the relative difference curve in order to show the quality of the glass containers produced at the forming station. In FIG. 7, the relative difference curve is shown for the glass container of FIG. 2 with the corresponding measurement-ratio curve shown in FIG. 4.

In this specific example the relative difference curve in FIG. 7 shows a positive deviation in the upper part of the glass container and a negative deviation in the lower part of the glass container, indicating too much glass in the upper part of the glass container and too little glass in the lower part of the glass container. The relative difference curve will be close to zero at every point for high quality glass containers.

Subsequently, the processor unit (44) compares the relative difference curve with predetermined tolerance curves and generates an alarm signal if a relative difference value exceeds the corresponding tolerance value. This is illustrated hereunder:

Alarm if:

$$\Delta I_{s,y} < I_{T-,y} \text{ or } \Delta I_{s,y} > I_{T+,y}$$

Where:

$I_{T-,y}$ = the negative tolerance value for line y; and

$I_{T+,y}$ = the positive tolerance value for line y.

The alarm signal may, for example, be used in order to reject glass containers which have an unacceptable quality on line 56 in FIG. 1. In FIG. 7 the negative tolerance values are set at -30% and the positive tolerance values are set at +30%. In FIG. 7 an alarm signal is generated because the relative difference values for line 300 through line 380 exceed the positive tolerance values.

In order to adjust the forming process automatically, the processor unit 44 may send the relative difference curve from each forming station to the control unit 38 over line 48. The control unit 38 adjusts the appropriate process parameters until the relative difference curve for each forming station is close to zero. This may be done without the need to have an operator monitoring the process continuously.

The processor unit 44 is synchronized with the forming machine 20 and with conveyor belt 50 in such a way that processor unit 44 knows from which forming station each glass container 30 originates.

Referring next to FIG. 8, the major components of the hot glass container quality analytical system of the present invention are illustrated in schematic fashion. An I.S. machine 60 has a conveyor 62 extending therefrom on which a stream of hot glass containers 64 are conveyed away from the I.S. machine 60. Two camera modules 66 and 68 are located in positions to monitor the hot glass containers 64 as they pass by the two camera modules 66 and 68 on the conveyor 62. In a preferred embodiment, the camera modules 66 and 68 each include a SWIR imaging acquisition device that scans a vertical line which will be used to acquire images of the hot glass containers 64 as they pass by on the conveyor 62. As the hot glass containers 64 pass by, a multiplicity of vertical lines will be scanned that will together form electronic images of the hot glass containers 64.

The first camera module 66 is located with an axis orthogonal to the longitudinal axis of the conveyor 62 on which the hot glass containers 64 travel after leaving the I.S. machine 60, with the conveyor 62 being oriented toward the side of the hot glass containers 64 passing by on the conveyor 62. The second camera module 68 is located with an axis at a predefined angle with respect to the longitudinal axis of the conveyor 62, with the conveyor 62 also being oriented toward the side of the hot glass containers 64 passing by on the conveyor 62. The camera modules 66 and 68 are preferably located such that their respective axes intersect at a point at the middle of the lateral axis of the conveyor 62, which is at the centerline of the conveyor the conveyor 62 (if they are not so located, appropriate mathematical compensation may be made). It should be noted that instead of the two camera modules 66 and 68 being used, either a single camera module 66 located as it is located in FIG. 8, or more than the two camera modules 66 and 68, could instead be used, although better overall results may be obtained through the use of more than a single camera module 66 being used.

The camera modules 66 and 68, which are the first of the major components of the hot glass container quality analytical system of the present invention, are connected to a control unit 70 that is used to operate the hot glass container quality analytical system of the present invention, which is the second of the major components of the hot glass container quality analytical system of the present invention. The camera module 66 is connected to the control unit 70 via a connection 72, and the camera module 68 is connected to the control unit 70 via a connection 74. The connections 72 and 74 may be network connections such as TCPIP network connections.

A user interface module 76, which is the third of the major components of the hot glass container quality analytical system of the present invention, is connected to the control unit 70 via a connection 78, which may be a network connection such as a TCPIP network connection. The user interface module 76 will be used both to display information generated by the hot glass container quality analytical system as well as to set up the hot glass container quality analytical system. Further, the information generated by the hot glass container quality analytical system and displayed on the user interface module 76 may be used to assess the quality of the hot glass containers 64 manufactured by the I.S. machine 60 as well as to control the operation of the I.S. machine 60 to improve the quality of the hot glass containers 64 manufactured by the I.S. machine 60.

Based upon the information generated by the hot glass container quality analytical system of the present invention, the hot glass containers 64 that are determined to be of unacceptable quality by the hot glass container quality analytical system are rejected and removed from the stream of the hot glass containers 64 on the conveyor 62. A glass container reject mechanism 80 that performs this function is operated by the control unit 70 via a connection 82, which may be a simple trigger signal such as a twenty-four Volt pulse.

The hot glass container quality analytical system of the present invention is provided with timing pulses by an I.S. machine control unit 84 via a connection 86. These timing pulses are used by the hot glass container quality analytical system to define from which section and which mold each of the hot glass containers 64 on the conveyor 62 originates. In this way, the hot glass container quality analytical system of the present invention can display this information to an operator accessing the user interface module 76. It is also contemplated that the information generated by the hot glass container quality analytical system of the present invention may be used to automatically control the I.S. machine 60 through the I.S. machine control unit 84 via the connection 86.

It may be noted that the control unit 70 of the hot glass container quality analytical system of the present invention is depicted as having another connection 88, the distal end of which is not shown as being connected in FIG. 8. This connection 88, which may also be a network connection such as a TCPIP network connection, may be used to connect additional remote control units (not shown in FIG. 8) which may be located, for example, in a control room at the glass container manufacturing plant at which the I.S. machine 60 is located, in an engineer's office either at that manufacturing plant or at another remote location, and/or in another remote location such as, for example, the hot glass container quality analytical system provider's facility to remotely monitor and troubleshoot the system at customer locations.

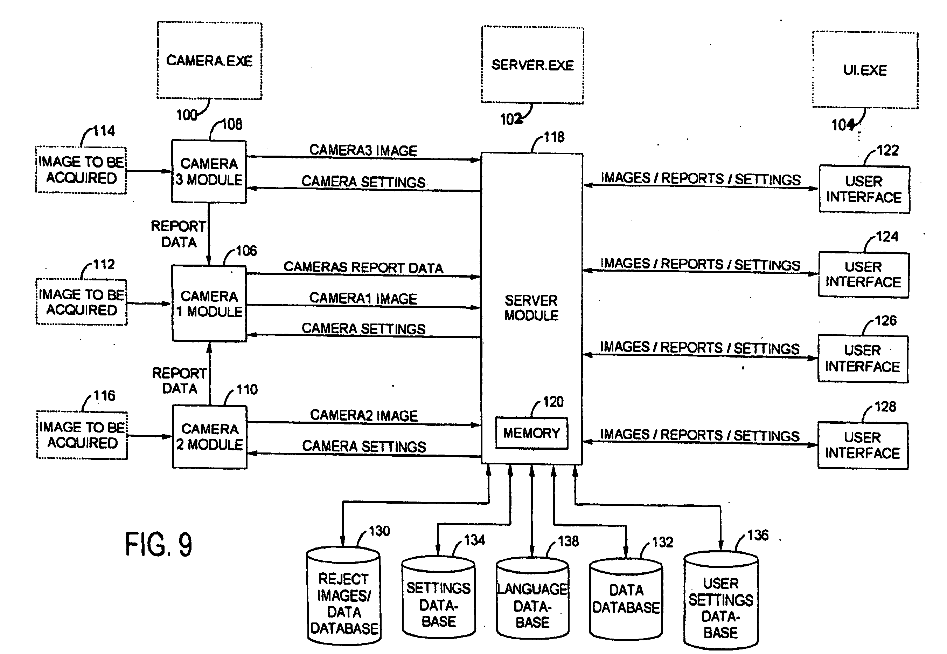

Referring next to FIG. 9, the operational connections of the essential elements of the exemplary hot glass container quality analytical system are shown in a manner that illustrates the flow of data between one or more camera modules, a server module, and one or more user interface modules. These three elements are networked together, typically by TCPIP network connections, which may be local or remote, or a combination of both local and remote. At a high level, the three essential components of the exemplary hot glass container quality analytical system that were referenced in FIG. 8 (the camera modules, the control unit, and the user interface module) are shown in an expanded fashion illustrating the implementation of three software executable modules, namely a camera software module 100, a server software module 102, and a user interface software module 104. These three executables may reside on separate computers, or, alternatively on the same computer. Alternately, the three software executable modules may be partially combined or fully combined into a single executable module.

In the implementation of FIG. 9, three camera modules 106, 108, and 110 are shown, of which it will be understood that first camera module 106 is the one that is mounted orthogonally to the conveyor (and which corresponds to the camera module 66 in FIG. 8). Thus, the first camera module 106 is used to acquire first images 112, the second camera module 108 is used to acquire second images 114, and the third camera module 110 is used to acquire third images 116 (all of which are obtained from different angles with respect to hot glass containers). It is entirely possible for the hot glass container quality analytical system of the present invention to accommodate inputs from at least four camera modules if desired.

The camera modules 106, 108, and 110 do calculations on the images 112, 114, and 116 respectively detected by an infrared camera contained in each of the camera modules 106, 108, and 110 and operating in a vertical line scanning manner as glass containers pass by the location of the infrared camera on the conveyor. The camera modules 106, 108, and 110 do calculations on the glass container images 112, 114, and 116, respectively, and each generates a glass container image report for each glass container image. The glass container image reports from the second camera module 108 and the third camera module 110 (if they are used) are provided to the first camera module 106, which is the main camera module, which consolidates the glass container image reports from the camera modules 106, 108, and 110 for each glass container.

The consolidated glass container image report is sent from the first camera module 106 to a server module 118, which contains memory 120. The memory 120 of the server module 118 is preferably at least sufficient to store all of the glass container reports for all glass containers monitored by the hot glass container quality analytical system of the present invention for the last hour. The images 112, 114, and 116 from the three camera modules 106, 108, and 110, respectively, are sent from the three camera modules 106, 108, and 110, respectively, to the server 118 (and these images 112, 114, and 116 pass from the camera modules 106, 108, and 110, respectively, through the server 118 to any of four user interface modules 122, 124, 126, and 128 if the user of these modules 122, 124, 126, and 128 wish to view them). Otherwise, the images 112, 114, and 116 are not sent from the camera modules 106, 108, and 110, respectively. The server 118 itself sends camera settings to each of the three camera modules 106, 108, and 110 to control the operation of the three camera modules 106, 108, and 110.

The user interface modules 122, 124, 126, and 128 are used to access the system and view screens presenting a wide array of information regarding the operation of the hot glass container quality analytical system, including live images, reports, and also screens allowing settings to be inputted into the hot glass container quality analytical system. Each of these user interface modules 122, 124, 126, and 128 is identical or essentially identical, with typical locations for the user modules 122, 124, 126, and 128 being on the plant floor, in a control room at the plant, in an engineer's office at the plant, and in remote locations such as the hot glass container quality analytical system provider's facility to remotely monitor and troubleshoot it at a customer's location. The user interface modules 122, 124, 126, and 128 typically include an interactive display such as a touchscreen or a mouse or trackpad interface in conjunction with a display screen.

The server module 118 also operates five different databases. The first of these databases is a rejected glass container images database 130, which in the preferred embodiment is used to contain images of at least the most recent 50,000 glass containers that have been rejected. The server module 118 calculates periodic averages for each cavity of the I.S. machine manufacturing the glass containers being analyzed. These periodic averages may be calculated as often as once each minute and as infrequently as once each 24 hours, with the preferred period being once every five minutes. These periodic averages for each cavity are stored in a second database, namely the glass container data database 132. Both the rejected glass container images database 130 and the glass container data database 132 may include time identifying data (the rejected glass container images database 130 includes the image of the rejected glass container, the time associated with each rejected glass container, and the reason that the glass container was rejected).

A third database, namely a parameter settings database 134, is used to store all of the parameters for a particular glass container, including camera settings, system settings, and limits for some parameters. A fourth database is a user settings database 136, which is used to control user interfaces through the user interface modules 122, 124, 126, and 128. The user settings database 136 may store preferred settings for each user for user passwords, user rights associated with each user, and language used by each user. A fifth database is a language database 138, which contains data needed to operate the user interfaces 122, 124, 126, and 128 in each of a plurality of languages useable with the hot glass container quality analytical system.

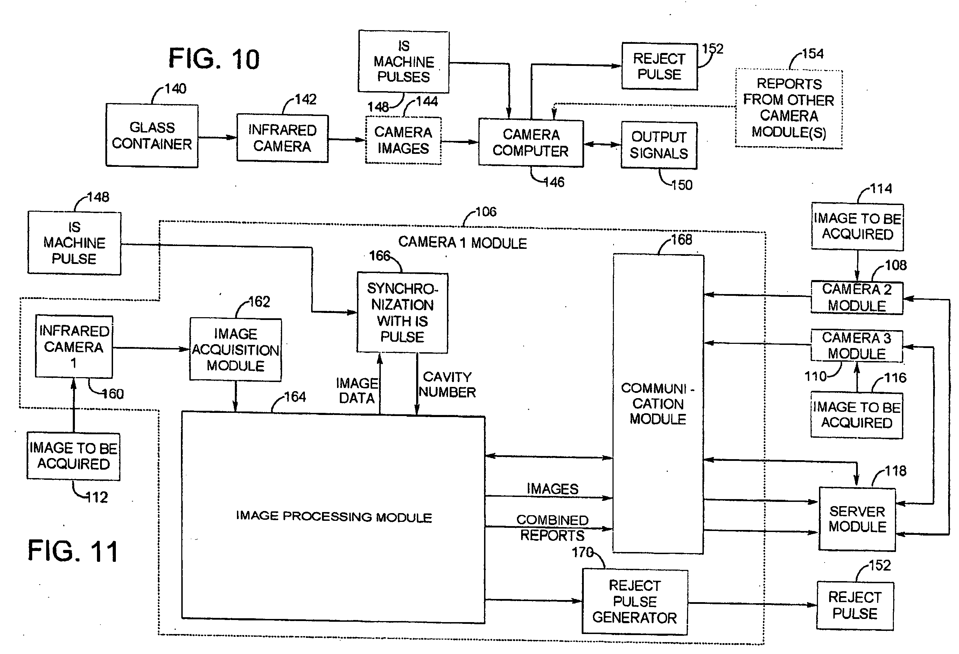

Referring next to FIG. 10, the operation of any of the camera modules illustrated in FIG. 9 is shown at a fairly high level. A glass container 140 is imaged by an infrared camera 142, which produces camera images 144 that are provided to a camera computer 146. As implemented in the preferred embodiment, the infrared camera 142 does not use a telecentric lens, which would have to be as large as the largest glass container to be viewed by the infrared camera 142 (since telecentric lenses collect rays in parallel rather than in the fashion of non-telecentric lenses). Thus, it will be appreciated that the distance of the infrared camera 142 from the glass containers is quite important in order for the system to operate properly.

The camera computer 146 extracts the image of each glass container 140 and also produces a glass container image report for each glass container 140. I.S. machine pulses 148 are provided to the camera computer 146, which is able to determine for each image of a glass container 140 in which section and mold the glass container it represents was manufactured. The camera computer 146 sends the glass container image report for each glass container image 140 together with the extracted images thereof as output signals 150 to the server module 118 (shown in FIG. 9). Alternately, if the camera module is an auxiliary camera module rather than the main camera module, it will send the glass container image report for each glass container 140 to the main camera module for consolidation into a combined glass container image report.

The infrared camera 142 also provides a reject pulse 152 for each glass container image 140 that it determines is of unacceptable quality. This reject pulse 152 is sent to the glass container reject mechanism 80 (shown in FIG. 8) to reject each unacceptable quality glass container. If the camera module is the main camera module, it will receive glass container image report(s) from one or more auxiliary camera modules 154 for each image of a glass container 140, which it will consolidate into a combined glass container image report that it sends to the server module 118.

Referring now to FIG. 11, the operation of the first camera module 106 illustrated in FIG. 9 is shown at a much more detailed level. The first images 112 are acquired by a first infrared camera 160 and provided to an image acquisition module 162 which receives the images of the stream of hot glass container 64 on the conveyor 62 (both shown in FIG. 8) and sends these continuous images to an image processing module 164 for further analysis. Each image will have a resolution of a number of horizontal lines and a number of vertical lines that is determined by the vertical resolution of the first infrared camera 160 and the scan frequency (the number of scans taken as the glass containers move along the conveyor 62 (also shown in FIG. 8). In one preferred embodiment, the resolution is 1024 horizontal lines and 1024 vertical lines.

The I.S. machine pulses 148 from the I.S. machine control unit 84 of the I.S. machine 60 (both shown in FIG. 8) are sent to a synchronization module 166 in the first camera module 106, where they are used to time the cycle of the hot glass container quality analytical system. There is one I.S. machine pulse 148 per complete I.S. machine cycle (which includes the manufacture of one hot glass container 64 from each of the cavities in each of the sections of the I.S. machine 60). This I.S. machine pulse 148 is used to establish where hot glass containers 64 from each of the sections and each of the cavities in each section are on the conveyor 62. The synchronization module 166 uses this data to identify in which of the sections and in which of the cavities in each of the sections each hot glass container 64 was molded. This information is then provided by the synchronization module 166 to the image processing module 164.

The image processing module 164 performs a variety of analyses on the images of the hot glass containers 64, which will be discussed after the operational construction of the hot glass container quality analytical system has been fully discussed herein. The image processing module 164 provides combined glass container reports and images obtained from the first infrared camera 160 to a communications module 168, which forwards this information to the server module 118. The glass container reports from the second camera module 108 and the third camera module 110 are provided to the communications module 168, which forwards them to the image processing module 164 on a data bus for combination with the glass container report generated in the image processing module 164 for images from the first infrared camera 160.

The images from the second camera module 108 and the third camera module 110 are provided to the server module 118. The image processing module 164 causes a reject pulse generator 170 to generate the reject pulse 152 used to reject any hot glass containers 64 of less than acceptable quality.

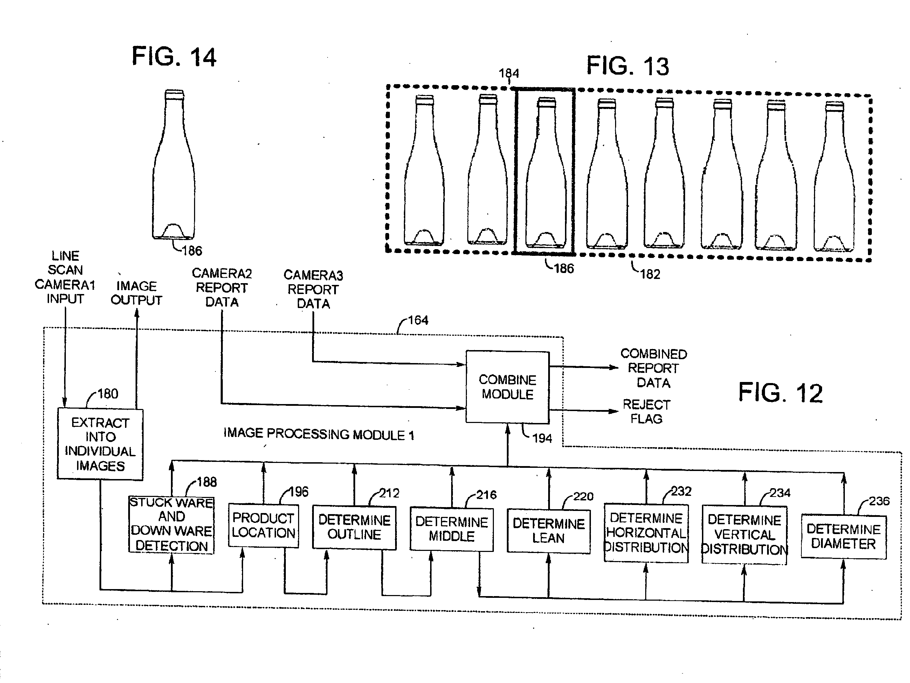

Referring next to FIG. 12, the functional details of the image processing module 164 of the first camera module 106 (shown in FIG. 11) are illustrated. The line scan input from the first camera is provided by the image acquisition module 162 to an image extraction module 180 which will extract individual images of each hot glass container 64. Referring to FIG. 13 in addition to FIG. 12, a digital "filmstrip" of images 182 is shown. The image extraction module 180 functions to obtain a frame of each hot glass container, such as the single hot glass container image frame 184 in which the single hot glass container image 186 is located, by using edge detection to determine the locations of large changes in intensity. It will be appreciated that the single hot glass container image 186, shown by itself in FIG. 14, consists of a selected number of horizontal lines and a selected number of vertical lines that are respectively determined by the vertical resolution of the first infrared camera 160 and the scan frequency.

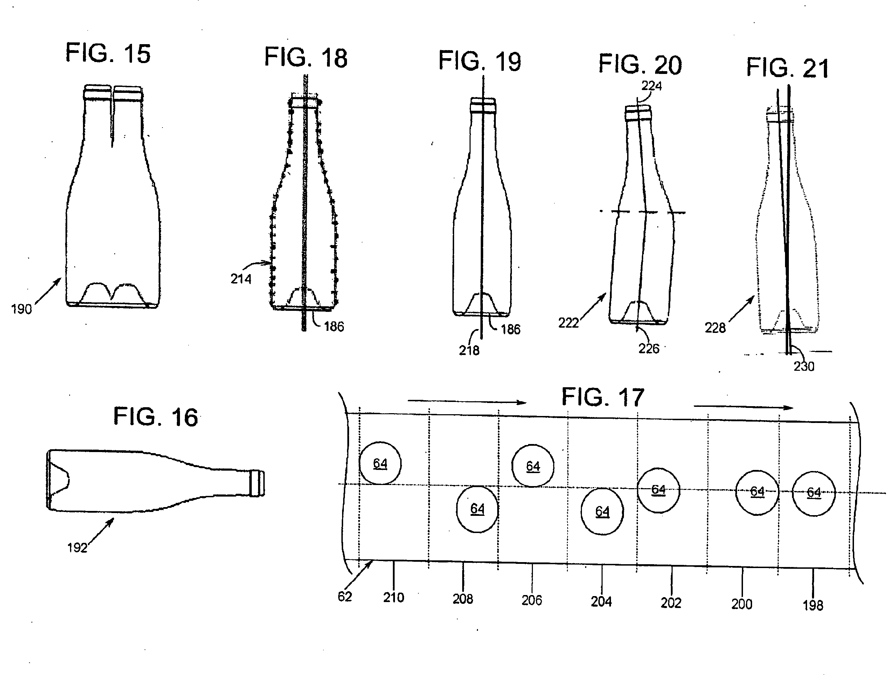

Once the single hot glass container image 186 has been extracted by the image extraction module 180, it may be analyzed by the remaining functional modules in the image processing module 164. In a stuck ware /down ware detection module 188, the single hot glass container image 186 is analyzed to determine whether it is either a "stuck ware," where two still-plastic hot glass containers 64 come into contact with each other and become stuck together as illustrated by the stuck ware 190 illustrated in FIG. 15. Such stuck ware 190 must necessarily be rejected. The stuck ware 190 is detected by using the outline of each hot glass container image 186, which was obtained by using edge detection as mentioned above. Once the outline of the hot glass container image 186 has been determined, a stuck ware 190 may be detected by determining the width of the hot glass container image 186, typically at a location in the body of the hot glass container 64 (which is typically the widest portion of the hot glass container 64). If the width is substantially larger than it should be (even approaching double the expected width), a stuck ware determination may be made and the hot glass container 64 may be rejected.

A similar situation is that of a "down ware," where a hot glass container 64 has fallen down, as illustrated by the down ware 192 illustrated in FIG. 16. This conditions is also identified by the dimensions of the images of the hot glass container image 186. The existence of either a stuck ware 190 or a down ware 192 is communicated to a combine module 194 that is used to combine all of the glass container report information. Additionally, if no hot glass container 64 is detected, a "missing" condition is determined and communicated to the combine module 194.

The next functional module in the image processing module 164 is a product location module 196, in which the location of the hot glass containers 64 on the conveyor 62 in seven consecutive longitudinal locations 198, 200, 202, 204, 206, 208, and 210 on the conveyor 62 is determined (shown in FIG. 17). Each glass container has an ideal longitudinal location and an ideal lateral location on the conveyor 62. The ideal lateral position on the conveyor 62 is along a line extending laterally on the conveyor (it may or may not be on the centerline of the conveyor 62, depending upon the size of the glass container being manufactured), a location that is defined as Y=0, and the ideal longitudinal position on the conveyor 62 is related to the I.S. machine pulse and for each hot glass container 64 is the desired longitudinal location on the conveyor 62 is defined as X=0.

The longitudinal offset of each hot glass container 64 is determined by the image from the first infrared camera 160 (shown in FIG. 11) in the first camera module 106 (shown in FIG. 9) only, and the lateral offset of each hot glass container 64 is determined by the image from both the first camera module 106 and at least one additional image, for example the image from the second camera module 108 (also shown in FIG. 9). The longitudinal and lateral offsets are used to modify the timing of the pushers that move the hot glass containers 64 from individual section deadplates adjacent to the conveyor 62 onto the conveyor 62. The timing of the operation of the pushers controls the longitudinal offset of the hot glass containers 64 on the conveyor 62, and the angular rotation of the mechanical pusher arms controls the lateral offset of the hot glass containers 64 on the conveyor 62.

The hot glass container 64 in the longitudinal location 198 is the only one of the seven hot glass containers 64 that is in the correct location, centered both longitudinally and laterally in the longitudinal location 198 (X=0, Y=0). The hot glass container 64 in the longitudinal location 200 is ahead of where it should be (X=+2, Y=0), the hot glass container 64 in the longitudinal location 202 is behind of where it should be (X=-2, Y=0), the hot glass container 64 in the longitudinal location 204 is across the centerline from where it should be (X=0, Y=+2), the hot glass container 64 in the longitudinal location 206 is behind the centerline from where it should be (X=0, Y=-2), the hot glass container 64 in the longitudinal location 208 is ahead of where it should be and across the centerline from where it should be (X=+2, Y=+2), and the hot glass container 64 in the longitudinal location 210 is behind of where it should be and behind the centerline from where it should be (X=-2, Y=-2). The longitudinal and lateral positions of each of the hot glass containers 64 is communicated to the combine module 194.

Next, the process moves to a determine outline module 212, in which the outline of the single hot glass container image 186 (shown in FIG. 14) is determined. The outline of each hot glass container image 186 is determined by using edge detection to determine the locations of large changes in intensity. The location of such large changes in intensity define the outline of the hot glass container 64. The detected outline 214 for the single hot glass container image 186 is illustrated in FIG. 18. The outline of the hot glass container 64 is communicated to the combine module 194.

Next, the process moves to a determine middle module 216, in which the middle of each hot glass container 64 is obtained by fitting a line to the middle of the outline of the hot glass container image 186. This may be performed by finding the midpoint of the portions of each of the horizontal lines beginning and ending at the edges of the hot glass container image 186. By determining the average of these midpoints, a best fit vertical line defining the middle 218 of each hot glass container 64 may be determined. Alternately, instead of using the average of these midpoints, the median midpoint of all of the horizontal lines beginning and ending at the edges of the hot glass container image 186 may instead be used. In either case, the middle 218 of each hot glass container 64 is communicated to the combine module 194.

The process now moves to a determine lean module 220, in which edge detection is used to determine the edges of the hot glass container image 186, and to find the midpoint of each of the horizontal lines beginning and ending at the edges of the hot glass container image 186. Referring now to FIG. 20, a "leaner" 222 is illustrated. The image of the "leaner" 222 is then divided into top and bottom halves, and a best fit algorithm is used to fit a line 224 through the top half and a line through the bottom half 226 of the "leaner" 222. Next, lean is calculated for each of the top half 224 and the bottom half 226 of the "leaner" 222, and lean is calculated for the entire "leaner" 222. Using these results, it can be determined if a hot glass containers 64 is a bent neck, a base leaner, or a bent neck base leaner. The top half lean 224 and the bottom half lean 226 of each hot glass container 64 is communicated to the combine module 194.

Referring briefly to FIG. 21, alternately, a single lean only can be calculated for a "leaner" 228. Again, edge detection is used to determine the edges of the hot glass container image 186, and to find the midpoint of each of the horizontal lines beginning and ending at the edges of the hot glass container image 186. The total lean 230 is thereby calculated for the "leaner" 228, and communicated to the combine module 194.

Referring again only to FIG. 12, the process now moves to a determine horizontal distribution module 232, in which the horizontal distribution of glass in the hot glass containers 64 is determined. Horizontal distribution uses the intensity of the horizontal scan lines on each hot glass container image 186. Once again, edge detection is used to determine the edges of the hot glass container image 186, and only the portions of the horizontal lines that are between the edged of the hot glass container image 186 are used.

The center of each horizontal line on the hot glass container image 186 is determined by having equivalent sums of digital values of pixels on each side thereof. Each pixel on each horizontal scan line has a digital value, and the center of horizontal distribution is the pixel that has a roughly equivalent total digital count (the sum of the digital values of each of the pixels) on each side of the center). This may be thought of as the median location on each of the horizontal scan lines, and has nothing to do with averaging. The offset of the center from the midpoint of each horizontal line is calculated. These offsets for each horizontal line are then divided by the width of the glass container at that horizontal line (the number of pixels in that horizontal line). The horizontal distribution may then be graphically displayed for the entire height of the glass container (this will be illustrated with reference to a screenshot below). The horizontal distribution information is communicated to the combine module 194.

The process now moves to a determine vertical distribution module 234, in which the vertical distribution of glass in the hot glass containers 64 is determined. (This is what was determined as the normalized measurement-ratio curve of FIG. 4.) The determination of vertical distribution begins with a determination of the line digital value measurement for each horizontal scan line on each hot glass container image 186. For each horizontal scan line on the single hot glass container image 186, this is the sum of the digital values of each of the pixels on the horizontal scan line.

The total digital value measurement, which is the sum of the digital values of all of the pixels on all of the horizontal scan lines on the glass container, is also determined. For each horizontal scan line, the line digital value measurement is divided by the total digital value measurement to normalize the vertical distribution determination to yield a measurement-ratio value for each horizontal scan line. This removes the distance from the molds and the resultant unequal cooling between the hot glass containers 64 as factors. Thus, the measurement-ratio values for each horizontal scan line are no longer intensities but are rather dimensionless numbers. By plotting these measurement-ratio values on the X-axis against the vertical position on each of the hot glass containers 64 as the Y-axis, the vertical distribution of the glass in each of the hot glass containers 64 may be indicated. The vertical distribution information is communicated to the combine module 194.

The process then moves to a determine diameter module 236 in which information regarding the diameter of the hot glass containers 64 is obtained. Diameter information may be determined either as the diameter of a selected line or as the diameter of a selected region, either of which result in a single diameter number, or as a diameter curve for the entire glass container. The line location or the region location is programmable in the hot glass container quality analytical system of the present invention. Also, multiple line locations or region locations may be used to check diameter at a number of different heights on the glass container.

For a multiple line region (which may be, for example, 20 lines), the median diameter value in the multiple line region is preferably used, although an average could instead be used if desired. If a diameter curve is to be obtained, the diameter is calculated for each horizontal scan line on the single hot glass container image 186, and such a diameter curve may be displayed as a curve (by plotting the calculated diameter values for each horizontal scan line on the X-axis against the vertical position on each of the hot glass containers 64 as the Y-axis). The diameter information is also communicated to the combine module 194.

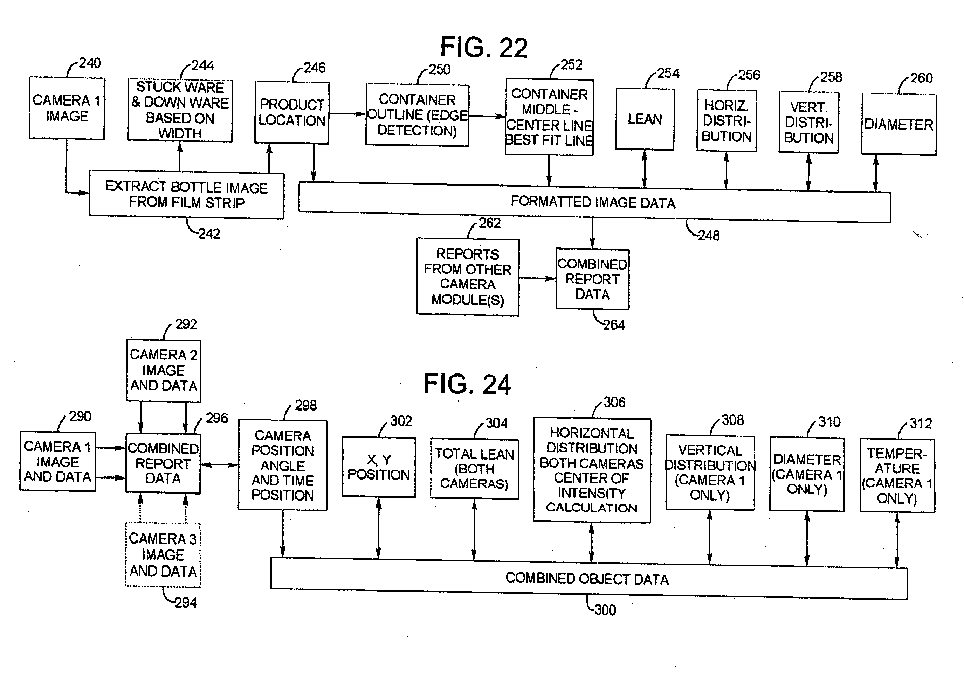

Referring next to FIG. 22, the flow of formatted data used to generate a combined report is illustrated. The image from a first camera image 240 is provided to an image extraction module 242, which extracts images of the hot glass containers 64. The images are provided to a stuck ware / down ware module 244 which will identify stuck ware and/or down ware based on width characteristics (and, in the case of down ware, height characteristics) of the image. The images are also provided to a product location module 246, which identifies the location of the hot glass containers 64 on the conveyor 62 (shown in FIG. 8). It will be appreciated that the product location module 246 must also have images obtained from another camera in order to provide both longitudinal and lateral displacement information regarding the hot glass containers 64 on the conveyor 62. Formatted image data regarding product location is supplied from the product location module 246 to a formatted image data bus 248.

The image information from the product location module 246 is also provided to an outline detection module 250, which uses edge detection to identify the outline of the glass containers. This data is provided to a center line determination module 252 that determines the best fit center line of the outline of the glass containers and provides formatted image data of the same to the formatted image data bus 248. A lean determination module 254 determines the lean of the outline of the glass containers and provides formatted image data of the same to the formatted image data bus 248.

A horizontal distribution determination module 256 determines the horizontal distribution of the outline of the glass containers and provides formatted image data of the same to the formatted image data bus 248. A vertical distribution determination module 258 determines the vertical distribution of the outline of the glass containers and provides formatted image data of the same to the formatted image data bus 248. A diameter determination module 260 determines the diameter of the outline of the glass containers and provides formatted image data of the same to the formatted image data bus 248. The formatted image data from the formatted image data bus 248 is used together with formatted image data from reports from other camera modules 262 to generate combined report data 264.

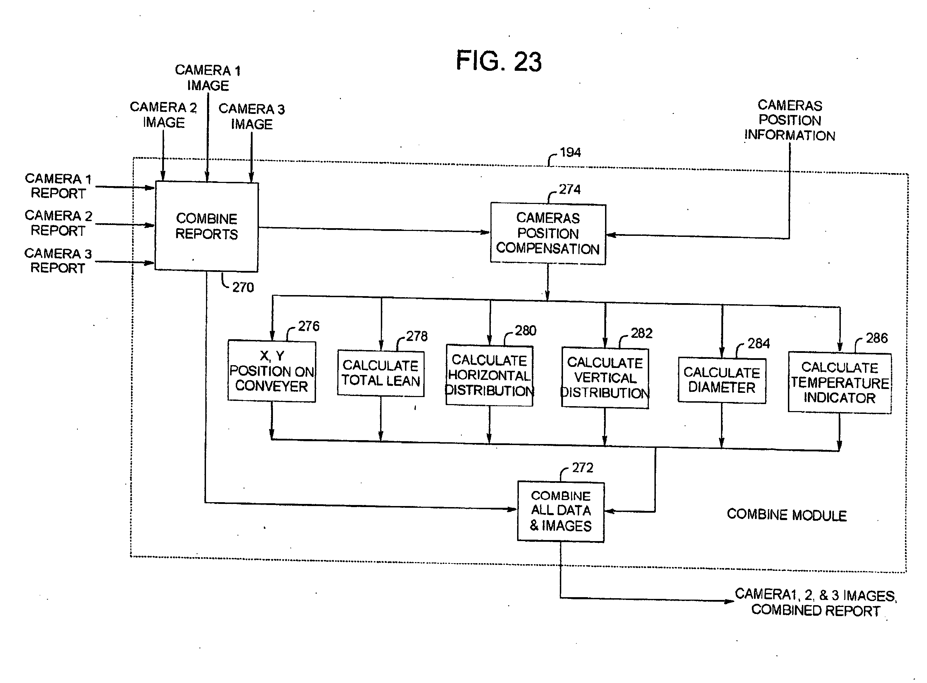

Referring now to FIG. 23, the combination of report data and images from a plurality of camera modules as inputs to the combine module 194 (FIG. 12) to produce the combined report and the images as outputs is illustrated. It will be understood that the images essentially flow through the combine module 194, although they may be annotated for display on user interface modules (not illustrated herein). The images from three cameras and the report data from three camera modules are provided as inputs to a combine reports module 270, which provides the images to a combine data and images module 272.

The report data is provided to a camera position compensation module 274, which also has as an input information defining the relative positions of the cameras. The camera position compensation module 274 provides its output to six modules that perform final calculations based upon the information contained in the three camera reports. An X, Y position calculation module 276 uses the information obtained from multiple reports to determine the longitudinal and lateral displacement of each hot glass container 64 on the conveyor 62, and provides this information to the combine data and images module 272. A total lean calculation module 278 uses the information obtained from multiple reports to determine the total lean of each hot glass container 64, and provides this information to the combine data and images module 272.

A horizontal distribution determination module 280 uses the information obtained from multiple reports to determine the horizontal distribution of each hot glass container 64, and provides this information to the combine data and images module 272. A vertical distribution calculation module 282 uses the information obtained from multiple reports to determine the vertical distribution of each hot glass container 64, and provides this information to the combine data and images module 272. A diameter calculation module 284 uses the information obtained from multiple reports to determine the diameter of each hot glass container 64, and provides this information to the combine data and images module 272. A temperature calculation module 286 uses the information obtained from multiple reports to determine the temperature of each hot glass container 64, and provides this information to the combine data and images module 272.

The manners in which the calculations identified in FIG. 23 are made is described with reference to FIG. 24. First camera image and data 290, second camera image and data 292, and third camera image and data 294 are combined to produce combined report data 296 which is interfaced with camera position angle and time data 298 to produce combined object data that is provided to a combined object data bus 300. However, in the following discussion of FIG. 24, is will be assumed that two cameras are used, and the third (shown in dotted lines) is optional and not used for the purposes of this discussion. An X, Y position determination module 302 uses determines the longitudinal and lateral displacement of each hot glass container 64 on the conveyor 62, and produces combined object data that is provided to a combined object data bus 300.

A total lean determination module 304 uses the lean calculations made for the hot glass container image for each of the images from the two cameras. Both the lean and the sign of the lean (positive or negative) is determined for each of the images from the cameras, and the leans and their signs are then used to calculate total lean taking into account the relative angular positions of the cameras. If the total lean for a glass container calculated in this manner is excessive, the glass container will be rejected for lean. The total lean determination module 304 produces combined object data that is provided to the combined object data bus 300.

A horizontal distribution determination module 306 uses the horizontal distribution calculations made for the hot glass container image for each of the images from the two cameras. It will be recalled that the offset of the center of intensity from the midpoint of each horizontal line in each hot glass container image was calculated, and then divided by the width of that hot glass container image at that horizontal line. In combining the horizontal distribution data from two camera images, the larger value for each horizontal line from the two camera images is used to determine the offset for that horizontal line. The horizontal distribution determination module 306 produces combined object data that is provided to the combined object data bus 300.

A vertical distribution determination module 308 preferably uses the vertical distribution calculations made for the hot glass container image from only one of the images from the two cameras (preferably from the first camera, which is located orthogonally with respect to the conveyor 62 (FIG. 8). Due to the nature of vertical distribution, it is not believed to be necessary to use the vertical distribution calculations made for the hot glass container images from both of the two cameras, although the calculations for each horizontal line could instead be averaged. The vertical distribution determination module 308 produces combined object data that is provided to the combined object data bus 300.

A diameter determination module 310 preferably uses the diameter calculations made for the hot glass container image from only one of the images from the two cameras (preferably from the first camera, which is located orthogonally with respect to the conveyor 62 (FIG. 8). Due to the nature of the diameter, it is not believed to be necessary to use the diameter calculations made for the hot glass container images from both of the two cameras, although the calculations for diameters from the two cameras could instead be compared and/or averaged on a horizontal scan line by horizontal scan line basis. In a preferred embodiment, if the two diameters differ an indication is provided that the glass container is not round, and if the difference is too large the glass container would be rejected. The diameter determination module 310 produces combined object data that is provided to the combined object data bus 300.

A temperature determination module 312 is used to provide an indication of temperature by determining the sum of the digital values of all of the pixels on all of the horizontal scan lines on the image of each glass container. Due to the nature of the temperature, it is not believed to be necessary to use the temperature calculations made for the hot glass container images from both of the two cameras, although the calculations could instead be averaged. In addition, a plot of the median values by mold may also be made, for example from the coolest mold (the furthest away) to the hottest mold (the closest), with a best fit line being plotted from the median values. The temperature determination module 312 produces combined object data that is provided to the combined object data bus 300.

In monitoring the hot glass containers as they stream from the I.S. machine where they are molded, the philosophy of the hot glass container quality analytical system of the present invention differs from that of merely checking each glass container against some arbitrary "perfect" glass container standard. Instead, the initial assumption is made that at least eighty percent of the glass containers being manufactured are of acceptable quality, and that less than twenty percent of the glass containers being manufactured are of lesser quality. The objective of the hot glass container quality analytical system of the present invention is to correct that twenty percent. Thus, the overriding objective of the hot glass container quality analytical system is not to make glass containers "perfect" according to some pre-defined standard, but rather to make them consistently so that they are all essentially the same. In order to do so, the aim is to present information to the operator of the I.S. machine that identifies outliers, and thereby assists the operator to adjust the I.S. machine to bring the outliers more into conformity with the others.

In this regard, many of the characteristics of each hot glass container that are determined by the hot glass container quality analytical system of the present invention are compared with a baseline value that is determined according in a novel manner. The values of each determined characteristic for each hot glass container that are determined by the hot glass container quality analytical system are stored on a first in, first out manner for a predetermined time period that may be varied. In a preferred embodiment, that characteristic is thirty minutes, although it could be varied from as little as one minute to as much as twenty-five hours, or even longer.

An example of the use of the median from the last thirty minutes (the predetermined period) is the calculation of the diameter at a particular location on the hot glass containers. When the diameter value at a particular location on a hot glass container or the diameter curve for a hot glass container has been determined, it can be compared with a value or a curve that is the median value (or a curve that contains the median values of each horizontal scan line) determined over the last thirty minutes, which may be shown as a solid line or a solid curve on the display.

From the values for each determined characteristic for each hot glass container for the predetermined period, the median values for each characteristic at that point in time are selected, and the determined characteristics for the current hot glass container may be compared with these median characteristics. It will be appreciated that the determination of the median characteristics occurs with every hot class container, since the predetermined period (e.g., thirty minutes) will change for each consecutive glass container. The median characteristics may be visually displayed (typically as a solid line) together with the image of the hot glass container. This is an important distinction from the operation of European Patent Application No. EP 09075545.5, filed on December 10, 2009, and assigned to the assignee of the present patent application.

An important advantage of the hot glass container quality analytical system of the present invention is that since it normalizes the image information for the hot glass containers, it can render unnecessary the determination of different medians for some of the characteristics of hot glass containers coming from different sections or molds that are based upon temperature variations of the hot glass containers coming from these different sections and molds. For example, consider the determination of vertical distribution of glass in the hot glass containers. Since information derived from the image for each hot glass container for vertical distribution is normalized by dividing the line radiation measurement for each image line by the total radiation measurement, differences occurring due to unequal cooling of the hot glass containers was eliminated.

Thus, each determined characteristic for each hot glass container may be compared with its median value for the predetermined period (e.g., thirty minutes), and information may be provided that may accompany the visual display of the image of that hot glass container. Additionally, determinations of warnings or rejections of that hot glass container may be made based upon how much the determined characteristics vary from the median values for those determined characteristics. For example, a percentage deviation (positive or negative) from the median value for each determined characteristic may be set, so that any hot glass container that exceeds this percentage deviation for any determined characteristic will be rejected. Further, two lower percentage deviations (such as, for example, one-third and two-thirds of the percentage deviation required for rejection) may be cause for a warning to be presented or an alarm to be raised for the determined characteristic for the hot glass container. Alternately, the levels for each of warnings, alarms, and rejects may be individually selectable. Information regarding rejections, alarms, or warnings may also be visually displayed for each glass container, as will become apparent below in conjunction with the discussion of the screenshots.

An example of a manner in which determined characteristics may be displayed for a particular mold may be provided for determined temperature, the process for which determines the sum of the digital values of all of the pixels on all of the horizontal scan lines on a hot glass container. The median temperature indicator for all of the hot glass containers coming from each mold for the last half hour may be used as a comparison value. Thus, the temperature indicator for each hot glass container is compared to the median temperature indicator for the hot glass containers coming from the same mold for the last half hour (the predetermined period), with a percentage difference being indicated. In addition, a plot of the median values by mold may also be made, for example from the coolest mold (the furthest away) to the hottest mold (the closest), with a best fit line being plotted from the median values. Preferably, for the temperature determination, only one camera is used, preferably the first camera that is mounted orthogonally with respect to the conveyor.

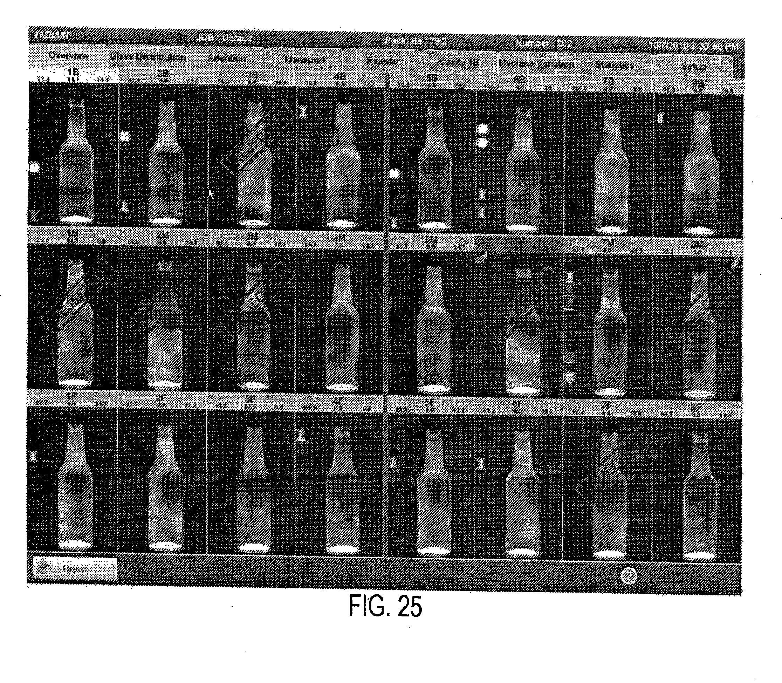

A number of screenshots that would be displayed on a touchscreen user interface module are also provided. Referring first to FIG. 25, an Overview screen is shown for an eight section, three mold I.S. machine. The screen is arranged to show a hot glass container from each of the molds in each of the sections, and is a real-time display of hot glass containers as they are monitored and analyzed by the hot glass container quality analytical system of the present invention. An image of each of the hot glass containers is both displayed and updated in real time.

In the preferred embodiment, the images are displayed in color using a color code keyed to the digital value representative of the radiation at each pixel of the images. For example, the hottest regions can be displayed as red, moving to orange, yellow, green, blue, and dark blue as the digital value of a pixel drops. The background of the containers, where the digital values are the lowest since the background is relatively cool compared to the hot glass bottle, may be arbitrarily displayed as black, as shown in FIG. 25, or, alternately, as white for purposes of contrast with the hot glass containers.

It may be seen that a number of the images of hot glass containers have been prominently labeled as rejected, and warnings are displayed on a number of other images of hot glass containers. At the top of the screen it may be seen that there are a number of tabs that may be used to access various screens contained in the hot glass container quality analytical system of the present invention. Since the user interface module is preferably touchscreen, a user can touch any of these tabs to access the other screens. By touching the ? icon in the lower right of the screen, a help screen for the currently displayed screen may be accessed. By touching the Reject button in the lower left corner, the hot glass container quality analytical system can be enabled to reject hot glass containers that meet the reject criteria, or to allow all of the hot glass containers to pass through on the conveyor.

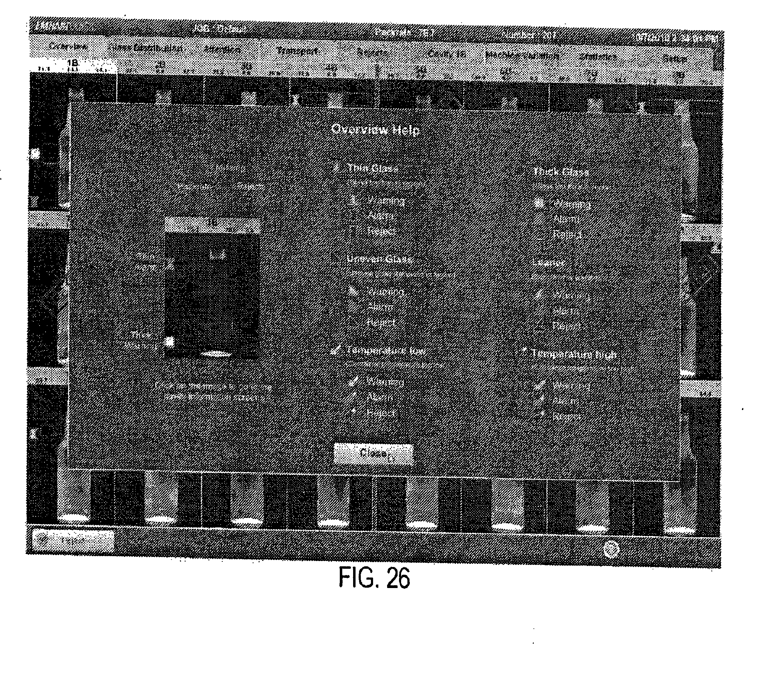

Referring next to FIG. 26 in conjunction with FIG. 25, the Overview Help screen is shown as being superimposed upon the Overview screen of FIG. 25. The Overview Help screen provides a legend for the various icons that may be used in the Overview screen. Unique icons are provided for thin glass, thick glass, uneven glass, leaners, and high or low temperatures. Three icons are provided for each of these categories of problems, with the icons preferably being colored yellow for warnings, orange for alarms, and red for rejections. Also the location of information on packrate, missing glass containers, and rejects for each mold is highlighted.

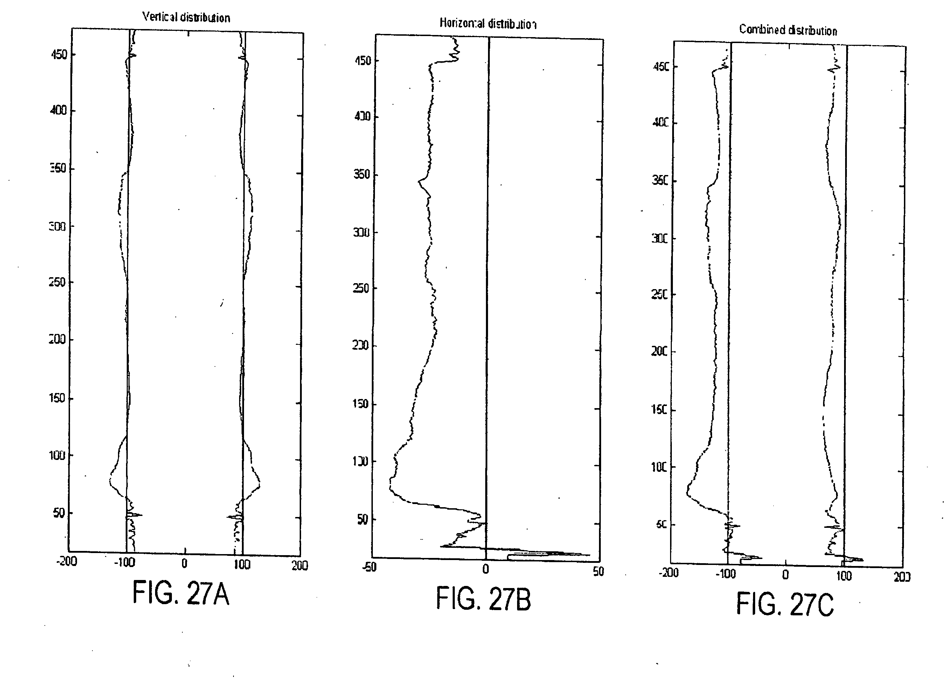

One of the innovations of the hot glass container quality analytical system of the present invention is that vertical glass distribution and horizontal glass distribution may be shown for a hot glass container in unified fashion. In the past, vertical glass distribution has been presented as a curve. (The present invention is also capable of presenting horizontal glass distribution as a curve. Referring now to FIG. 27A, the vertical glass distribution is presented as a solid plot rather than as a curve.) The solid plot is shown with respect to a rectangle which is the height of the vertical glass distribution curve and which has a volume representative of the amount of glass that should be contained in each of the hot glass containers.

The vertical glass distribution curve is placed over the right side of the rectangle with its average point(s) overlying the right side of the rectangle, and with its mirror image placed over the left side of the rectangle with the mirror image's average point (s) overlying the left side of the rectangle. The volume contained within the vertical glass distribution plot lying between these vertical glass distribution curves should be the same as the volume of the rectangle (assuming that the hot glass container contains the proper volume of glass). Thus, by looking at the visual depiction displayed in the vertical glass distribution plot, one can quickly understand the vertical glass distribution in the hot glass container.

Referring next to FIG. 27B, a horizontal glass distribution curve is illustrated for the same hot glass container. The horizontal glass distribution curve, of course, is plotted along a vertical line indicating the middle of the hot glass container, and ideally would coincide with that vertical line. To the extent that it does not, the horizontal glass distribution curve thus illustrates an unequal horizontal distribution of glass in the hot glass container at the locations at which it diverges from the vertical line. The horizontal glass distribution curve illustrated in FIG. 27B shows an improper distribution to the left.

Referring now to FIG. 27C, the horizontal distribution curve has been added to each of the vertical glass distribution curve and its mirror image, thereby resulting in a glass distribution plot that combines the vertical glass distribution curve and the horizontal distribution curve together into a single plot. A user looking at the glass distribution plot of FIG. 27C can clearly understand both the vertical glass and the horizontal distribution of glass in the hot glass container. This represents a significant advancement in the presentation of glass distribution information.

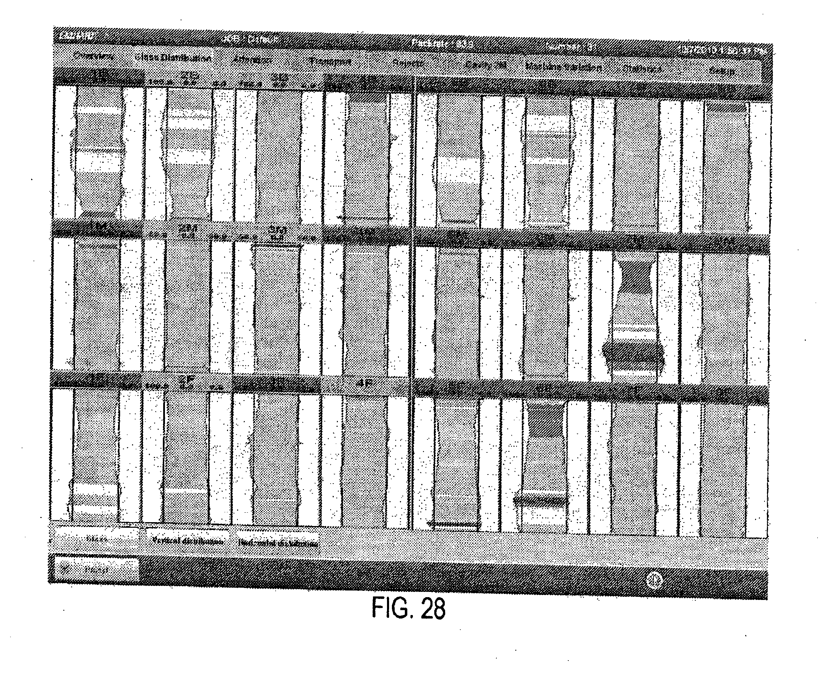

Referring now to FIG. 28, a Glass Distribution screen is shown for an eight section, three mold I.S. machine. It will be appreciated that the areas of each glass distribution plot that are either too thick or too thin, or in which the horizontal distribution is improper will be illustrated by horizontal bands of color. The bands of color may be different for warnings, alarms, and rejections, if desired (and as shown in FIG. 28). Like the Overview screen illustrated in FIG. 25, image of each of the glass distribution curves for each of the hot glass containers from each of the molds in each of the sections is both displayed and updated in real time.

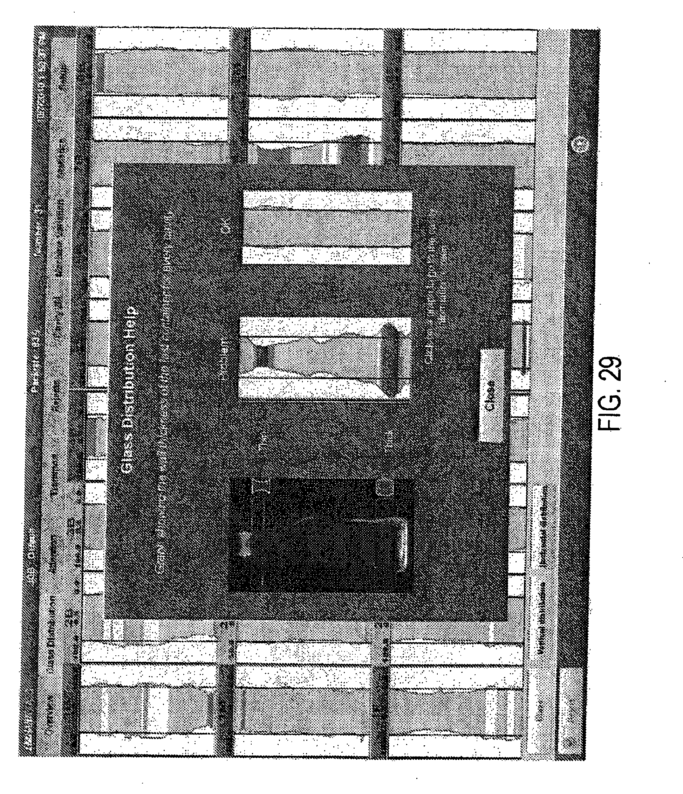

FIG. 29 is Glass Distribution Help screen that is shown as being superimposed upon the Glass Distribution screen of FIG. 28. The Glass Distribution Help screen provides a legend for the thick and thin icons that are used in the Glass Distribution screen. A problem glass distribution display as well as an acceptable glass distribution display are also shown in the Glass Distribution Help screen.

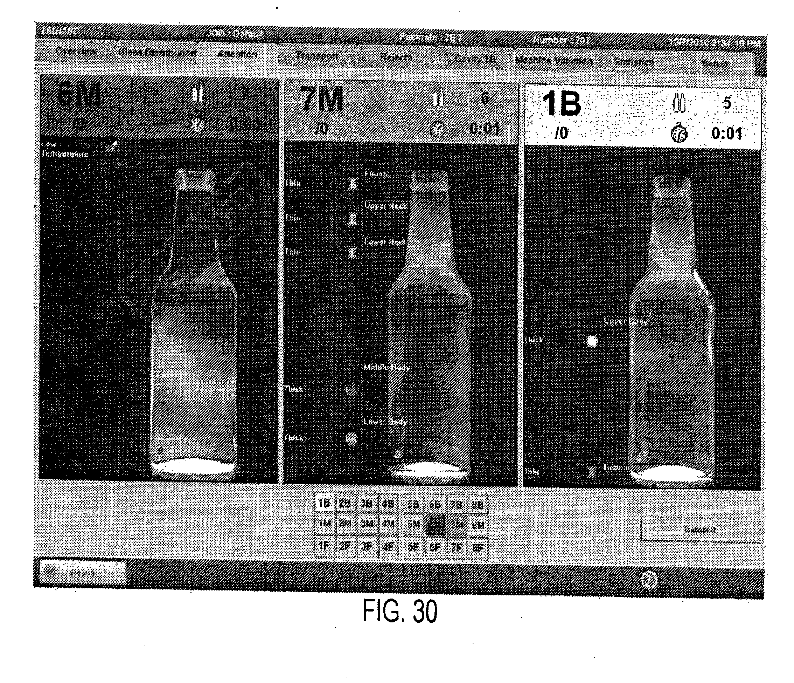

Referring next to FIG. 30, an Attention screen is shown which shows the three worst producing molds of the I.S. machine (over the last predetermined period, e.g., thirty minutes). In the preferred embodiment, the relative quality of the worst three are identified by the color of the band at the top of each of the three images of the hot glass containers, with red being the worst glass container, orange being the second worst glass container, and yellow being the third worst glass container. The location of each of the three worst hot glass containers is also shown in a schematic illustration showing the sections and molds.

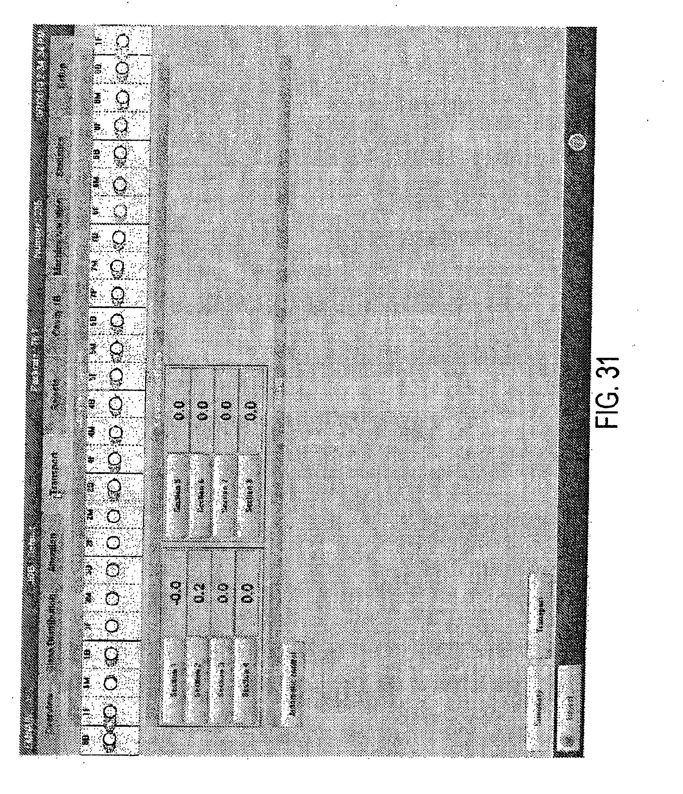

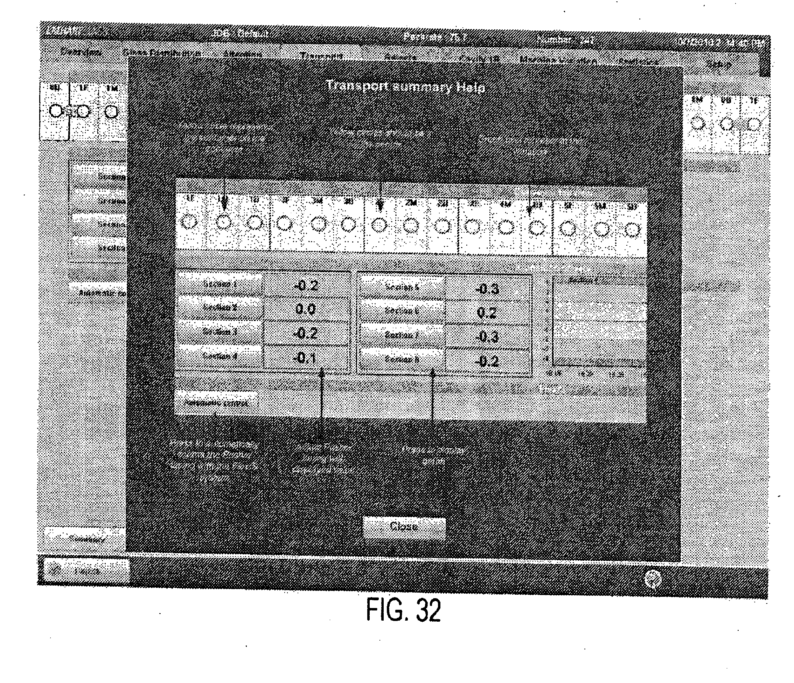

Referring now to FIG. 31, a Transport screen is shown that depicts the real time location of the last hot glass containers from each section and mold on the conveyor as well as the variation in positions over a selected time period. FIG. 32 is Transport Help screen that is shown as being superimposed upon the Transport screen of FIG. 31. The location of each hot glass container is shown by the circle, with variations in the locations of hot glass containers for the last predetermined time (e.g., thirty minutes) being shown by the shaded areas around the circles. Adjustments to the movement of the pusher mechanisms (the pusher mechanism pushes glass containers formed in the I.S. machine from a dead plate to the conveyor) may also be made, which will control to some degree the positions of the hot glass containers on the conveyor. The positions of a hot glass container from each mold in each section is both displayed and updated in real time in the Transport screen.

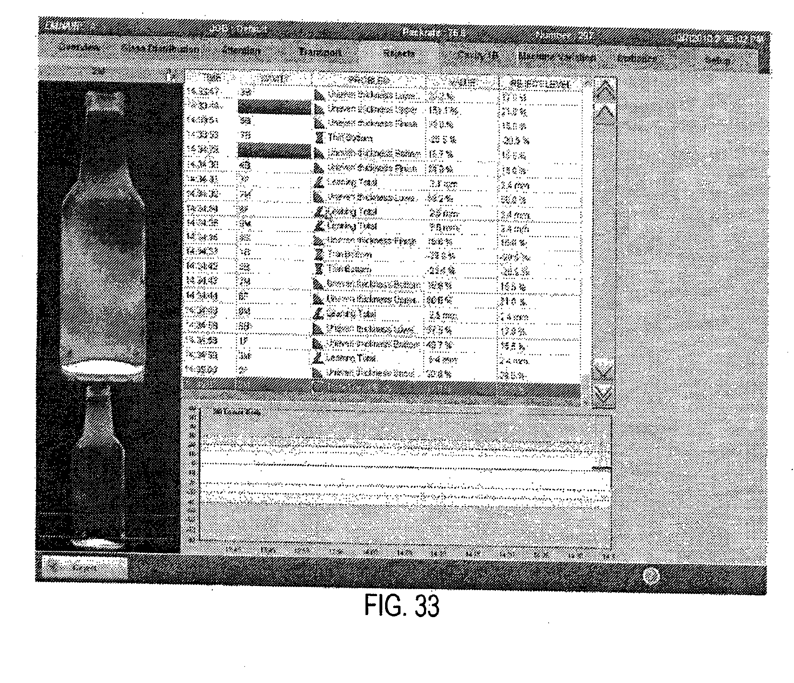

Referring next to FIG. 33, a Reject screen is shown which shows data regarding hot glass containers that have been rejected, including the particular problem resulting in the rejection. The data is shown as being arranged by time of rejection.

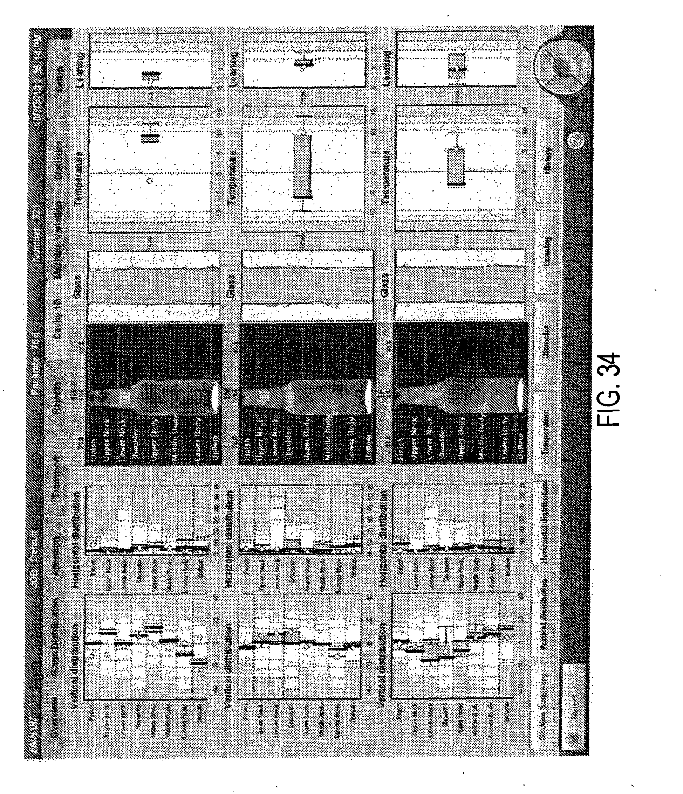

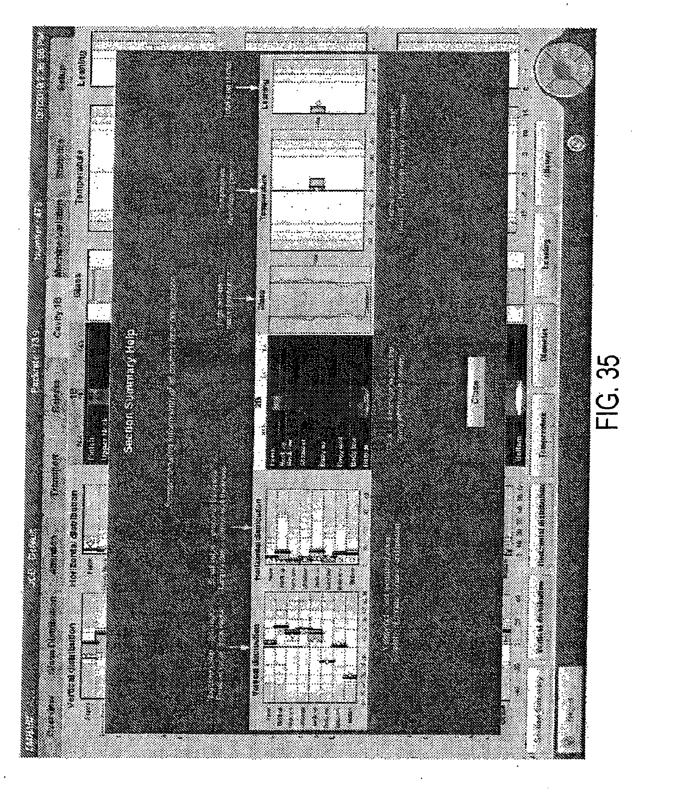

Referring now to FIGS. 34 and 35, a Section Summary screen is shown for a particular section in FIG. 34, and in FIG. 35 a Section Summary Help screen is shown as being superimposed upon the Section Summary screen of FIG. 34. The Section Summary screen shows a variety of information for the section, including Vertical Glass Distribution, Horizontal Glass Distribution, Glass Distribution, Temperature, and Leaning. For Vertical Glass Distribution, Horizontal Glass Distribution, Temperature, and Leaning, the circle (dot) represents the most recent hot glass container from the section and mold, and the boxplots show the variation for the last predetermined period (e.g., thirty minutes) as well as the median value for that predetermined period.

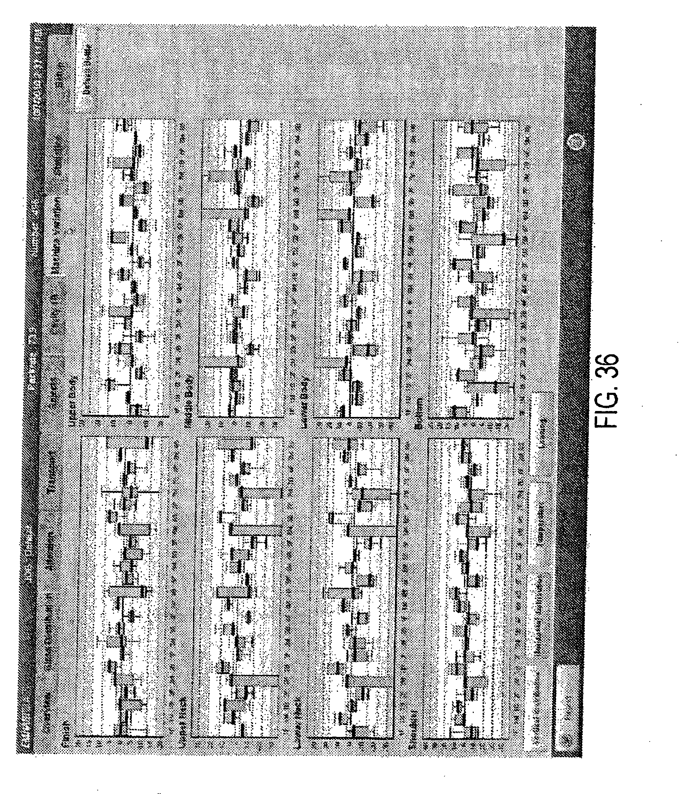

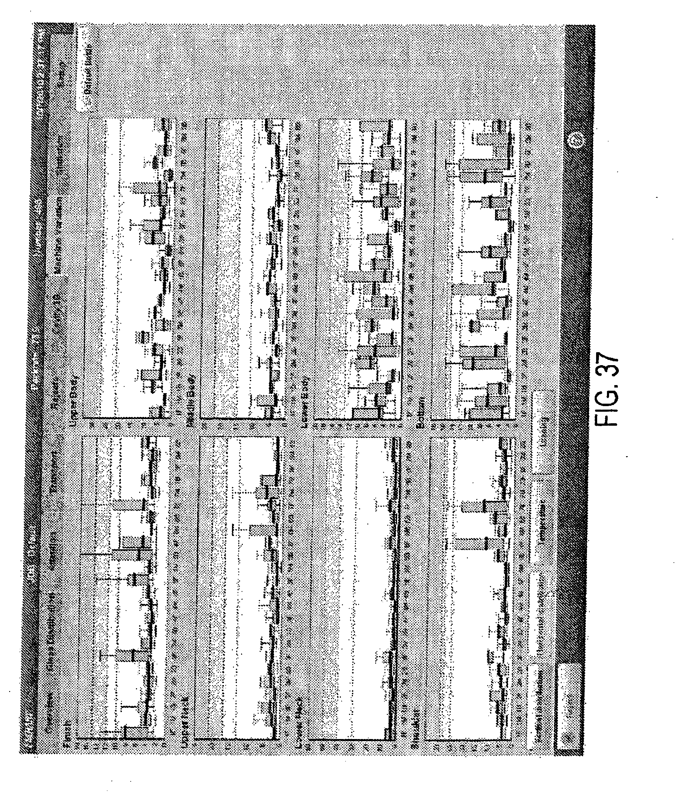

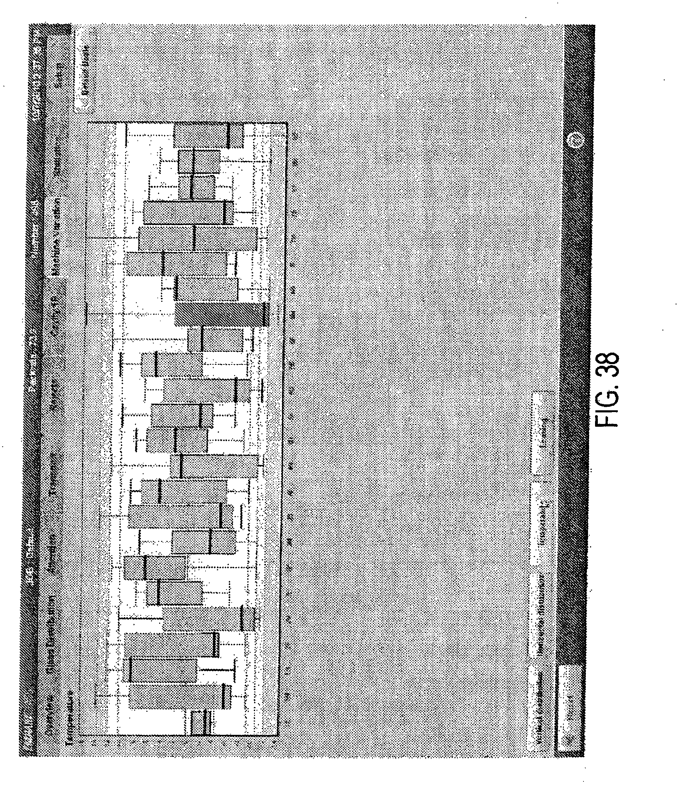

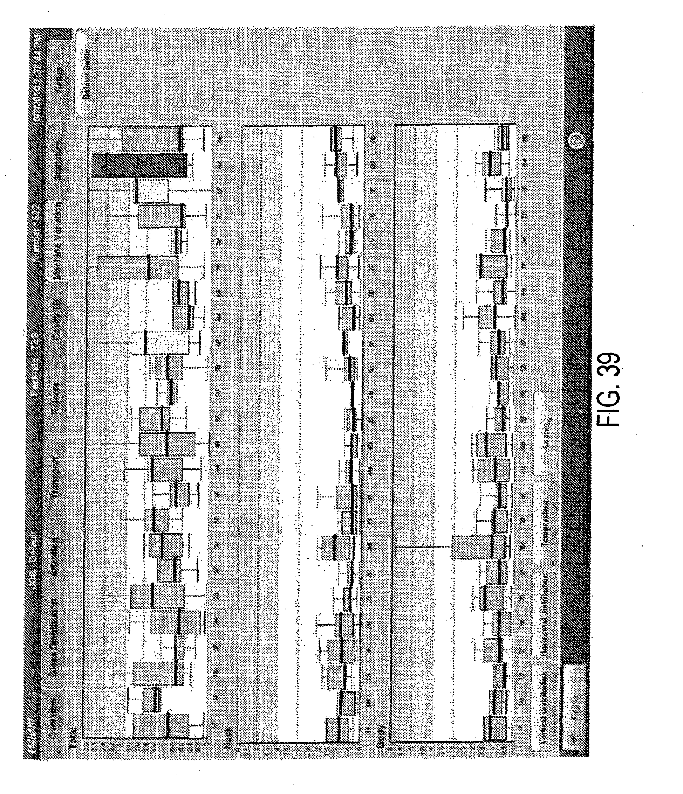

Referring next to FIGS. 36, 37, 38, and 39, Machine Variation screens are shown for Vertical Glass Distribution, Horizontal Glass Distribution, Temperature, and Leaning, respectively, for each of eight regions in the hot glass containers from the top to the bottom thereof. The boxplots again show the variation for the last predetermined period (e.g., thirty minutes) as well as the median value for that predetermined period.

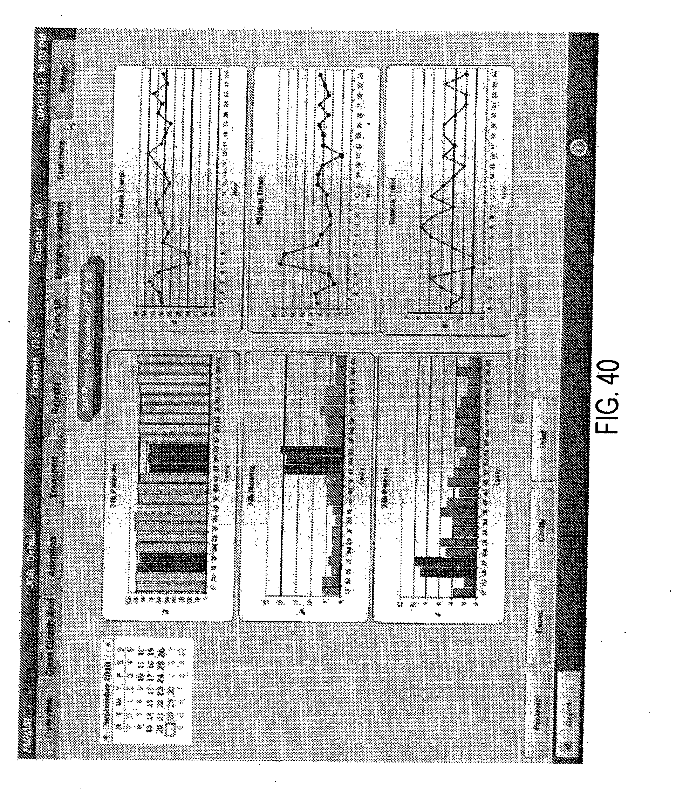

Finally, FIG. 40 shows a Statistics screen displaying summary information for the I.S. machine in graphical form for a predetermined period. This screen is particularly useful for daily production meetings, and can have its period of data collection set appropriately for such a meeting.

Although the foregoing description of the hot glass container quality analytical system of the present invention has been shown and described with reference to particular embodiments and applications thereof, it has been presented for purposes of illustration and description and is not intended to be exhaustive or to limit the invention to the particular embodiments and applications disclosed. It will be apparent to those having ordinary skill in the art that a number of changes, modifications, variations, or alterations to the invention as described herein may be made, none of which depart from the scope of the present invention. The particular embodiments and applications were chosen and described to provide the best illustration of the principles of the invention and its practical application to thereby enable one of ordinary skill in the art to utilize the invention in various embodiments and with various modifications as are suited to the particular use contemplated. All such changes, modifications, variations, and alterations should therefore be seen as being within the scope of the present invention as determined by the appended claims when interpreted in accordance with the breadth to which they are legally entitled.

## Claims

1. A method for monitoring and analyzing characteristics of hot glass containers (30,64) formed by an I.S. machine, the method comprising:

   monitoring, with at least one imaging device (42;66,68), radiation emitted by hot glass containers (30,64) immediately after they are formed and before they are cooled as they are conveyed from the I.S. machine on a conveyor (50,62);
   extracting an individual image of each of the hot glass containers (30,64) from the monitored radiation emitted by the hot glass containers (30,64);
   **characterized in that** the method performs the steps of:

   analyzing each individual extracted image to identify the presence or lack of deviations in the glass container forming process in each of a plurality of characteristics of the hot glass containers (30,64) by comparing each characteristic of the hot glass container (30,64) with the median one of the values for that characteristic in a plurality of hot glass containers (30,64) monitored during a predetermined time period immediately preceding the hot glass container (30,64) being evaluated;

displaying one or more of the individual extracted images of the hot glass containers (30,64) in real time on a display; and

simultaneously displaying on the display diagnostic information representative of one or more of the characteristics of the or each hot glass container (30,64) corresponding to the or each individual extracted image shown on the display and indicative of the presence or lack of a deviation in the at least one characteristics from the median value for those characteristics.

2. A method as claimed in Claim 1, wherein the at least one imaging device comprises:

a Short Wave Infrared (SWIR) camera.

3. A method as claimed in Claim 1, wherein the at least one imaging device comprises:

first and second imaging devices (66,68) positioned directly after the I.S. machine on opposing sides of and at different angles with respect to the conveyor (62) and to the hot glass container (64) being monitored.

4. A method as defined in Claim 1, wherein the analyzing step comprises:

determining the dimensional outline of each hot glass container (30,64) from the individual extracted images of the hot glass container (30,64); and

analyzing the dimensional outline of the hot glass container (30,64) to determine whether the hot glass container (30,64) is "stuck ware", "down ware", or "missing".

5. A method as defined in Claim 1, wherein the analyzing step comprises:

determining the dimensional outline of each hot glass container (30,64) from the individual extracted images of the hot glass container (30,64); and

analyzing the dimensional outline of the hot glass container (30,64) to determine any lean in the hot glass container (30,64);

wherein the lean of the glass container (30,64) is displayed on the display as diagnostic information.

6. A method as defined in Claim 1, wherein the analyzing step comprises:

determining the vertical distribution of glass in each hot glass container (30,64) from the individual extracted images of the hot glass container (30,64);

wherein the vertical distribution of glass in the glass container (30,64) is displayed on the display as diagnostic information.

7. A method as defined in Claim 1, wherein the analyzing step comprises:

determining the horizontal distribution of glass in each hot glass container (30,64) from the individual extracted images of the hot glass container (30,64);

wherein the vertical distribution of glass in the glass container (30,64) is displayed on the display as diagnostic information.

8. A method as defined in Claim 1, wherein the analyzing step comprises:

determining the vertical distribution of glass in each hot glass container (30,64) from the individual extracted images of the hot glass container (30,64);

determining the horizontal distribution of glass in each hot glass container (30,64) from the individual extracted images of the hot glass container (30,64);

wherein the vertical distribution and the horizontal distribution of glass in the glass container (30,64) are both displayed on the display as diagnostic information as a single display element.

9. A method as defined in Claim 1, wherein the analyzing step comprises:

setting at least one programmable horizontal region on the images of the hot glass containers (30,64) at a selected height on the images of the hot glass containers (30,64), the programmable horizontal region having a programmable height of at least one horizontal scan line;

determining a diameter of each hot glass container (30,64) at each programmable horizontal region from the individual extracted images of the hot glass container (30,64); and

providing at least one of the determined diameter or information relating to the presence or lack of a deviation in the determined diameter for the hot glass container (30,64) to the display.

10. A method as defined in Claim 9, wherein the at least one imaging device comprises:

first and second imaging devices (66,68) positioned directly after the I.S. machine on opposing sides of and at different angles with respect to the conveyor (62) on which the hot glass containers (64) are conveyed from the I.S. machine;

determining a diameter of each hot glass container (64) at each programmable horizontal region from the individual extracted images of the hot glass container (64) from each of the first and second imaging devices (66,68); and

if the diameters determined from the first and second imaging devices vary more than a predetermined amount for a hot glass container (64), providing an alarm or warning or rejecting the hot glass container (64).

11. A method as defined in Claim 1, wherein the analyzing step comprises:

assigning a color to each of a plurality of ranges of radiation emitted by hot glass containers (30,64);

generating a color image from each extracted image of the hot glass containers (30,64) by assigning the color associated with the range of radiation for each pixel of the individual extracted images of the hot glass containers (30,64);

wherein the displaying step comprises displaying the color image generated from the individual extracted images of the hot glass containers (30,64).

12. A method as defined in Claim 1, wherein the I.S. machine has a determined number of sections and a determined number of molds in each section, wherein the analyzing step comprises:

determining temperature diagnostic information representative of the temperatures of each of the hot glass containers (30,64) by calculating the sum of the digital values of all of the pixels on all of a plurality of horizontal scan lines on the image of each glass container (30,64);

determining, for each mold, the median value of the temperature diagnostic information representative of the temperatures of each of the hot glass containers (30,64) for a predetermined period stored in a first in, first out database;

plotting the median values by mold from the coolest mold to the hottest mold), and plotting a best fit line from the median values; and

determining, for each mold, the difference between the temperature diagnostic information representative of the temperatures of each of the hot glass containers (30,64) and the best fit line at the location of the same mold.

13. A method as defined in Claim 1, wherein the I.S. machine has a determined number of sections and a determined number of molds in each section, wherein the displaying step comprises:

simultaneously displaying an individual extracted image of a hot glass container (30,64) formed in each of the molds in each of the sections on the display, and updating the displayed individual extracted image of a hot glass container formed in each of the molds in each of the sections in real time.

14. A method as defined in Claim 1, wherein the I.S. machine has a determined number of sections and a determined number of molds in each section, wherein the displaying step comprises:

simultaneously displaying the three individual extracted images of the hot glass containers (30,64) from the three molds having the greatest deviations in the at least one characteristics.

15. A method as defined in Claim 1, wherein the analyzing step comprises:

   determining the location of each of the hot glass containers (30,64) on the conveyor (30,64); and

   wherein the position of each of the hot glass containers (30,64) is displayed on the display.

16. A method as defined in Claim 1, additionally comprising:

   storing diagnostic information representative of one or more of the characteristics of each of the hot glass containers (30,64) for a predetermined period in a first in, first out database;
   determining the median of the diagnostic information representative of one or more of the characteristics of each of the hot glass containers (30,64) for a predetermined period stored in the first in, first out database; and
   comparing the diagnostic information representative of one or more of the characteristics of each of the hot glass containers (30,64) with the median.

17. A method as defined in Claim 16, wherein a boxplot of the diagnostic information representative of one or more of the characteristics of each of the hot glass containers (30,64) for a predetermined period stored in the first in, first out database and its median are displayed together with the diagnostic information representative of the one or more of the characteristics of each of the hot glass containers (30,64) on the display.

18. A method as defined in Claim 16, wherein if the comparison of the diagnostic information representative of one or more of the characteristics of a hot glass container (30,64) with the median exceeds a predetermined percentage, an alarm or warning is provided or the hot glass container (30,64) is tejected.

19. A method as defined in Claim 1, wherein the diagnostic information simultaneously displayed on the display in a section summary mode comprises:

   at least three of Vertical Glass Distribution, Horizontal Glass Distribution, Glass Distribution, Temperature, and Leaning.

20. A method as defined in Claim 1, additionally comprising:

   using the diagnostic information to automatically control the forming process in the I.S. machine.

21. A method as defined in Claim 1, wherein the display comprises:

   a touchscreen user interface.

22. A system for monitoring and analyzing characteristics of hot glass containers (30,64) formed by an I.S. machine, the system comprising:

   at least one imaging device (42;66,68) which monitors the radiation emitted by hot glass containers (30,64) immediately after they are formed and before they are cooled as they are conveyed from the I.S. machine on a conveyor (50,62);
   an image extraction module (190,242) that extracts an individual image of each of the hot glass containers (30,64) from the monitored radiation emitted by the hot glass containers (30,64);
   **characterized by**:

   an image processing module (164) that analyzes each individual extracted image to identify the presence or lack of deviations in the glass container (30,64) forming process in each of a plurality of characteristics of the hot glass containers (30,64) by comparing each characteristic of the hot glass container (30,64) with the median one of the values for that characteristic in a plurality of hot glass containers (30,64) monitored during a predetermined time period immediately preceding the hot glass container (30,64) being evaluated; and
   a display on which at least one of the individual extracted images of the hot glass containers (30,64) is displayed in real time; wherein diagnostic information representative of at least one of the characteristics of the or each hot glass container (30,64) corresponding to the or each individual extracted image shown on the display and indicative of the presence or lack of a deviation in the at least one characteristics from the median values for those characteristics is simultaneously displayed on the display.

23. A system as defined in Claim 22, wherein the at least one imaging device comprises:

a Short Wave Infrared (SWIR) camera.

24. A system as defined in Claim 22, wherein the at least one imaging device comprises:

first and second imaging devices (66,68) positioned directly after the I.S. machine on opposing sides of and at different angles with respect to the conveyor (62) and to a hot glass container (64) when positioned to be monitored.

25. A system as defined in Claim 22, wherein the image processing module (164) comprises:

an outline determination module that determines the dimensional outline of each hot glass container (30,64) from the individual extracted images of the hot glass container (30,64); and
a module that analyzes the dimensional outline of the hot glass container (30,64) to determine whether the hot glass container (30,64) is "stuck ware", "down ware", or "missing".

26. A system as defined in Claim 22, wherein the image processing module (164) comprises:

an outline determination module (212,250) that determines the dimensional outline of each hot glass container (30,64) from the individual extracted images of the hot glass container (30,64); and
a lean determination module (220,254) that analyzes the dimensional outline of the hot glass container (30,64) to determine any lean in the hot glass container (30,64);

wherein the lean of the glass container (30,64) is displayed on the display as diagnostic information.

27. A system as defined in Claim 22, wherein the image processing module (164) comprises:

a vertical distribution determination module (234,258) that determines the vertical distribution of glass in each hot glass container (30,64) from the individual extracted images of the hot glass container (30,64);

wherein the vertical distribution of glass in the glass container (30,64) is displayed on the display as diagnostic information.

28. A system as defined in Claim 22, wherein the image processing module (164) comprises:

a horizontal distribution determination module (232,256) that determines the horizontal distribution of glass in each hot glass container (30,64) from the individual extracted images of the hot glass container (30,64);

wherein the horizontal distribution of glass in the glass container (30,64) is displayed on the display as diagnostic information.

29. A system as defined in Claim 22, wherein the image processing module (164) comprises:

a vertical distribution determination module (234,258) that determines the vertical distribution of glass in each hot glass container (30,64) from the individual extracted images of the hot glass container (30,64);
a horizontal distribution determination module (232,256) that determines the horizontal distribution of glass in each hot glass container (30,64) from the individual extracted images of the hot glass container (30,64);

wherein the vertical distribution and the horizontal distribution of glass in the glass container are both displayed on the display as diagnostic information as a single display element.

30. A system as defined in Claim 22, wherein the image processing module (164) comprises:

a database containing at least one programmable horizontal region on the images of the hot glass containers at a selected height on the images of the hot glass containers, the programmable horizontal region having a programmable height of at least one horizontal scan line; and

a diameter determination module (236,260) that determines a diameter of each hot glass container (30,64) at each programmable horizontal region from the individual extracted images of the hot glass container (30,64);

wherein diagnostic information representative of at least one of the determined diameter or information relating to the presence or lack of a deviation in the determined diameter for the hot glass container (30,64) is provided to the display.

31. A system as defined in Claim 30, wherein the at least one imaging device comprises:

first and second imaging devices (66, 68) positioned directly after the I.S. machine on opposing sides of and at different angles with respect to the conveyor (62) on which the hot glass containers (30, 64) are conveyed from the I.S. machine;

wherein the diameter determination module (236, 260) determines a diameter of each hot glass container (30, 64) at each programmable horizontal region from the individual extracted images of the hot glass container (30, 64) from each of the first and second imaging devices (66, 68); and

wherein if the diameters determined from the first and second imaging devices (66, 68) vary more than a predetermined amount for a hot glass container (30, 64), an alarm or warning is provided or the hot glass container (30, 64) is rejected.

32. A system as defined in Claim 22, wherein the image processing module (164) assigns a color to each of a plurality of ranges of radiation emitted by hot glass containers (30, 64), and wherein the image processing modulel (164) generates a color image from each extracted image of the hot glass containers (30, 64) by assigning the color associated with the range of radiation for each pixel of the individual extracted images of the hot glass containers (30, 64); and

wherein the color images generated from the individual extracted images of the hot glass containers (30, 64) are displayed on the display.

33. A system as defined in Claim 22, wherein the I.S. machine has a determined number of sections and a determined number of molds in each section, wherein the image processing module (164) determines temperature diagnostic information representation of the temperatures of each of the hot glass containers (30, 64) by calculating the sum of the digital values of all of the pixels on all of the horizontal scan lines on the image of each glass container;

wherein the image processing module (164) determines, for each mold, the median value of the temperature diagnostic information representative of the temperatures of each of the hot glass containers for a predetermined period stored in the first in, first out database;

wherein the image processing module (164) plots the median values by mold from the coolest mold to the hottest mold), and plots a best fit line from the median values; and

wherein the image processing module (164) determines, for each mold, the difference between the temperature diagnostic information representative of the temperatures of each of the hot glass containers (30, 64) and the best fit line at the location of the same mold.

34. A system as defined in Claim 22, wherein the I.S. machine has a determined number of sections and a determined number of molds in each section, wherein the display simultaneously displays an individual extracted image of a hot glass container (30, 64) formed in each of the molds in each of the sections on the display, and the displayed individual extracted image of a hot glass container (30, 64) formed in each of the molds in each of the sections is updated in real time.

35. A system as defined in Claim 22, wherein the I.S. machine has a determined number of sections and a determined number of molds in each section, wherein the display simultaneously displays the three individual extracted images of the hot glass containers (30, 64) from the three molds having the greatest deviations in the at least one characteristics.

36. A system as defined in Claim 22, wherein the image processing module (164) comprises:

a product location module (246) for determining the location of each of the hot glass containers (30, 64) on the conveyor; and

wherein information representative of the position of each of the hot glass containers (30, 64) is provided to the display.

37. A system as defined in Claim 22, wherein the image processing module (164) comprises:

a first in, first out database that stores diagnostic information representative of one or more of the characteristics of each of the hot glass containers (30, 64) for a predetermined period;
wherein the image processing module (164) determines the median of the diagnostic information representative of one or more of the characteristics of each of the hot glass containers (30, 64) for a predetermined period stored in the first in, first out database, and compares the diagnostic information representative of one or more of the characteristics of each of the hot glass containers (30, 64) with the median.

38. A system as defined in Claim 37, wherein a boxplot of the diagnostic information representative of one or more of the characteristics of each of the hot glass containers (30, 64) for a predetermined period stored in the first in, first out database and its median are displayed together with the diagnostic information representative of the one or more of the characteristics of each of the hot glass containers (30, 64) on the display.

39. A system as defined in Claim 37, wherein if the comparison of the diagnostic information representative of one or more of the characteristics of a hot glass container (30, 64) with the median exceeds a predetermined percentage, an alarm or warning is provided or the hot glass container (30, 64) is rejected.

40. A system as defined in Claim 22, wherein the display has a section summary mode in which at least three of Vertical Glass Distribution, Horizontal Glass Distribution, Glass Distribution, Temperature, and Leaning are simultaneously displayed on the display.

41. A system as defined in Claim 22, additionally comprising:

an I.S. machine control unit (84) that uses the diagnostic information to automatically control the forming process in the I.S. machine.

42. A system as defined in Claim 22, wherein the display comprises:

a touchscreen user interface.

**Patentansprüche**

1. Verfahren zum Überwachen und Analysieren von Eigenschaften heißer Glasbehälter (30, 64), die von einer IS-Maschine geformt wurden, durch
Überwachen der von heißen Glasbehältern 30, 64) abgegebenen Strahlung unmittelbar nach der Formgebung und vor dem Abkühlen während des Abführens aus der IS-Maschine auf einem Förderer (50, 61), wobei das Überwachen mittels mindestens einer Abbildungseinrichtung (42; 66, 68) erfolgt, und
Extrahieren eines Einzelbildes jedes heißen Glasbehälters (30, 64) aus der von ihm abgegebenen und überwachten Strahlung;
**gekennzeichnet** t durch folgende Schritte:

Analyse jedes extrahierten Einzelbildes auf das Vorliegen bzw. Fehlen von Abweichungen im Glasbehälter-Formungsprozess in jeder einer Vielzahl von Eigenschaften der heißen Glasbehälter (30, 64) durch Vergleichen jeder Eigenschaft der heißen Glasbehälter (30, 64) mit dem Median eines der Werte für diese Eigenschaft in einer Vielzahl von heißen Glasbehältern (30, 64) unmittelbar vor der Auswertung der während eines vorbestimmten Zeitintervalls überwachten heißen Glasbehälter (30, 64);
Darstellen eines oder mehr der extrahierten Einzelbilder der heißen Glasbehälter (30, 64) in Echtzeit auf einem Sichtfeld (Display); und
gleichzeitiges Ausgeben auf dem Sichtfeld von diagnostischen Informationen zu einer oder mehr der Eigenschaften des oder jedes der heißen Glasbehälter (30, 64) für das oder jedes der auf dem Sichtfeld dargestellten extrahierten Einzelbilder, wobei die Bildausgabe auf das Vorliegen bzw. Fehlen einer Abweichung in der mindestens einen Eigenschaft vom Medianwert für diese Eigenschaften verweist.

**2.** Verfahren nach Anspruch 1, bei dem die mindestens eine Abbildungseinrichtung eine kurzwellige Infrarot-Kamera (SWIR) aufweist.

**3.** Verfahren nach Anspruch 1, bei dem die mindestens eine Abbildungseinrichtung eine erste und eine zweite Abbildungsvorrichtung (66, 68) aufweist, die unmittelbar hinter der IS-Maschine auf gegenüberliegenden Seiten des und unter unterschiedlichen Winkeln zum Förderer (62) und den überwachten heißen Glasbehältern (64) angeordnet sind.

**4.** Verfahren nach Anspruch 1, bei dem der Analyseschritt beinhaltet:

Ermitteln des Abmessungsumrisses jedes der heißen Glasbehälter (30, 64) aus deren einzelnen extrahierten Einzelbildern; und
Analyse des Abmessungsumrisses des heißen Glasbehälters (30, 64) zum Ermitteln, ob der heiße Glasbehälter (30, 64) an einem anderen Behälter haftet, umgefallen ist oder fehlt.

**5.** Verfahren nach Anspruch 1, bei dem der Analyseschritt beinhaltet:

Ermitteln des Abmessungsumrisses jedes der heißen Glasbehälter (30, 64) aus deren extrahierten Einzelbildern; und
Analyse des Abmessungsumrisses des heißen Glasbehälters (30, 64) zum Ermitteln, ob er schief steht; wobei der Schiefstand des Glasbehälters (30, 64) als diagnostische Informationen auf dem Sichtfeld dargestellt wird.

**6.** Verfahren nach Anspruch 1, bei dem der Analyseschritt beinhaltet:

Ermitteln der vertikalen Verteilung des Glases in jedem der heißen Glasbehälter (30, 64) aus deren extrahierten Einzelbildern;
wobei die vertikale Glasverteilung in den Glasbehältern (30, 64) als diagnostische Information auf dem Sichtfeld dargestellt wird.

**7.** Verfahren nach Anspruch 1, bei dem der Analyseschritt beinhaltet:

Ermitteln der horizontalen Verteilung des Glases in jedem der heißen Glasbehälter (30, 64) aus deren extrahierten Einzelbildern;
wobei die vertikale Glasverteilung in den Glasbehältern (30, 64) als diagnostische Information auf dem Sichtfeld dargestellt wird.

**8.** Verfahren nach Anspruch 1, bei dem der Analyseschritt beinhaltet:

Ermitteln der vertikalen Verteilung des Glases in jedem der heißen Glasbehälter (30, 64) aus deren extrahierten Einzelbildern, und
Ermitteln der horizontalen Verteilung des Glases in jedem der heißen Glasbehälter (30, 64) aus deren extrahierten Einzelbildern;
wobei sowohl die vertikale als auch die horizontale Glasverteilung in den Glasbehältern (30, 64) auf dem Sichtfeld in Form eines einzigen Darstellungselements als diagnostische Information ausgegeben werden.

**9.** Verfahren nach Anspruch 1, bei dem der Analyseschritt beinhaltet:

Setzen mindestens eines programmierbaren horizontalen Bereichs auf den Bildern der heißen Glasbehälter (30, 64) in einer gewählten Höhe derselben, wobei der programmierbare horizontale Bereich eine programmierbare Höhe von mindestens einer horizontalen Abtastzeile aufweist;
Ermitteln eines Durchmessers jedes der heißen Glasbehälter (30, 64) in jedem programmierbaren horizontalen Bereich aus den extrahierten Einzelbildern der heißen Glasbehälter (30, 64); und
Weitergabe mindestens entweder des ermittelten Durchmessers oder der Information zum Vorliegen bzw. Fehlen einer Abweichung des ermittelten Durchmessers für die heißen Glasbehälter (30, 64) oder beider an das Sichtfeld.

**10.** Verfahren nach Anspruch 9, bei dem die mindestens eine Abbildungseinrichtung aufweist:

eine erste und eine zweite Abbildungsvorrichtung (66, 68), die unmittelbar nach der IS-Maschine auf gegenüber liegenden Seiten zu dem Förderer (62) und unter unterschiedlichen Winkeln dazu angeordnet sind, wobei der Förderer die heißen Glasbehälter (64) aus der IS-Maschine abführt;

Ermitteln eines Durchmessers jedes heißen Glasbehälters (64) in jedem programmierbaren horizontalen Bereich aus deren extrahierten Einzelbildern aus der ersten und der zweiten Abbildungsvorrichtung (66, 68); und Auslösen eines Alarmsignals bzw. einer Warnung oder Abweisen heißer Glasbehälters (64), falls die aus der ersten und der zweiten Abbildungsvorrichtung ermittelten Durchmesser für diese um mehr als einen vorbestimmten Betrag variieren.

11. Verfahren nach Anspruch 1, bei dem der Analyseschritt beinhaltet:

Zuweisen einer Farbe an jeden einer Vielzahl von Bereichen der von den heißen Glasbehältern (30, 64) abgegebenen Strahlung; und
Erzeugen eines Farbbildes aus jedem extrahierten Bild der heißen Glasbehälter (30, 64), indem jedem Bildpunkt der einzelnen extrahierten Bilder der heißen Glasbehälter (30, 64) die zum Strahlungsbereich des jeweiligen Bildpunkts gehörende Farbe zugewiesen wird;
wobei der Sichtdarstellschritt beinhaltet, die aus den einzelnen extrahierten Bildern der heißen Glasbehälter (30, 64) erzeugten Bilder sichtbar darzustellen.

12. Verfahren nach Anspruch 1, bei dem die IS-Maschine eine bestimmte Anzahl von Abschnitten und in jedem derselben eine bestimmte Anzahl von Formen aufweist und der Analyseschritt beinhaltet:

Ermitteln von jeden der heißen Glasbehälter (30, 64) darstellenden diagnostischen Temperaturinformationen durch Berechnen der Summe der digitalen Werte sämtlicher Bildpunkte in allen einer Vielzahl horizontaler Abtastzeilen im Bild jedes Glasbehälters (30, 64);
für jede Form: Ermitteln des Medianwerts der die Temperaturen jedes der heißen Glasbehälter (30, 64) darstellenden diagnostischen Temperaturinformationen für einen vorbestimmten Zeitraum, die in einer FIFO-Datenbank abgespeichert sind;
Erstellen einer Kurve der Medianwerte für die einzelnen Formen von der kältesten Form zur heißesten Form sowie der aus den Medianwerten ermittelten Best-fit-Gerade; und
für jede Form: Ermitteln der Differenz zwischen der die Temperaturen jedes der heißen Glasbehälter (30, 64) darstellenden diagnostischen Temperaturinformationen und der Best-fit-Geraden am Ort der gleichen Form.

13. Verfahren nach Anspruch 1, bei dem die IS-Maschine eine bestimmte Anzahl von Abschnitten und in jedem Abschnitt eine bestimmte Anzahl von Formen aufweist und der Sichtdarstellschritt beinhaltet:

das gleichzeitige Darstellen eines extrahierten Einzelbildes eines in jeder der Formen jedes der Abschnitte ausgebildet heißen Glasbehälters (30, 64) auf dem Sichtfeld und Echtzeit-Aktualisieren des dargestellten extrahierten Einzelbildes eines in jedem der Formen jedes der Abschnitte ausgebildeten heißen Glasbehälters.

14. Verfahren nach Anspruch 1, bei dem die IS-Maschine eine bestimmte Anzahl von Abschnitten und in jedem Abschnitt eine bestimmte Anzahl von Formen aufweist und der Sichtdarstellschritt beinhaltet:

gleichzeitiges Darstellen der drei extrahierten Einzelbilder von heißen Glasbehältern (30, 64) aus den drei Formen mit der stärksten Abweichung in der mindestens einen Eigenschaft.

15. Verfahren nach Anspruch 1, bei dem der Analyseschritt beinhaltet:

Ermitteln des Orts jedes der heißen Glasbehälter (30, 64) auf dem Förderer (50, 62) und Darstellen der Lage jedes der heißen Glasbehälter (30, 64) auf dem Sichtfeld.

16. Verfahren nach Anspruch 1, das zusätzlich beinhaltet:

Abspeichern von eine oder mehr der Eigenschaften jedes der heißen Glasbehälter (30, 64) darstellenden diagnostischen Informationen für einen vorbestimmten Zeitraum in einer FIFO-Datenbank;
Ermitteln des Medianwerts der eine oder mehr der Eigenschaften jedes der heißen Glasbehälter (30, 64) darstellenden diagnostischen Informationen für einen vorbestimmten Zeitraum, die in der FIFO-Datenbank abgespeichert sind; und

Vergleichen der einen oder mehr der Eigenschaften jedes der heißen Glasbehälter (30, 64) darstellenden diagnostischen Informationen mit dem Medianwert.

17. Verfahren nach Anspruch 16, bei dem die für einen vorbestimmte Zeitraum geltenden und in der FIFO-Datenbank abgespeicherten diagnostischen Informationen für eine oder mehr der Eigenschaften jedes der heißen Glasbehälter (30, 64) sowie deren Medianwert gemeinsam mit den für eine oder mehr der Eigenschaften jedes der heißen Glasbehälter (30, 64) geltenden diagnostischen Informationen auf dem Sichtfeld als Kastengrafik (Boxplot) dargestellt wird.

18. Verfahren nach Anspruch 16, bei dem, falls ein Vergleich der eine oder mehr der Eigenschaften eines heißen Glasbehälters (30, 64) darstellenden diagnostischen Informationen mit dem Medianwert einen vorbestimmten Prozentwert übersteigt, ein Alarm oder eine Warnmeldung ausgelöst oder der heiße Glasbehälter (30, 64) abgewiesen wird.

19. Verfahren nach Anspruch 1, bei dem die in einer für einen Abschnitt geltenden zusammenfassenden Darstellung gleichzeitig dargestellten diagnostischen Informationen mindestens drei der Größen "vertikale Glasverteilung", "horizontale Glasverteilung", "Glasverteilung", "Temperatur" und "Neigung" beinhalten.

20. Verfahren nach Anspruch 1, das zusätzlich das Anwenden der diagnostischen Informationen zur selbsttätigen Steuerung des Formgebungsprozesses in der IS-Maschine beinhaltet.

21. Verfahren nach Anspruch 1, bei dem das Sichtfeld eine Benutzerschnittstelle mit berührungsempfindlichem Bildschirm aufweist.

22. System zum Überwachen und Analysieren von Eigenschaften von in einer IS-Maschine gebildeten heißen Glasbehältern (30, 64) mit:

mindestens einer Abbildungseinrichtung (62; 66, 68), die die von heißen Glasbehältern (30, 64) abgegebenen Strahlung unmittelbar nach der Formgebung und vor dem Abkühlen beim Abführen aus der IS-Maschine auf einem Förderer (50, 62) überwacht, und
einem Bildgewinnungsmodul (190, 242), das aus der von den heißen Glasbehältern (30, 64) abgegebenen Strahlung ein Einzelbild jedes dieser heißen Glasbehälter (30, 64) erzeugt;
**gekennzeichnet durch**:

ein Bildbearbeitungsmodul (164), das jedes extrahierte Einzelbild der heißen Glasbehälter (30, 64) auf Abweichungen in jeder einer Vielzahl von Eigenschaften der heißen Glasbehälter (30, 64) im Formgebungsprozess überwacht, indem es jede Eigenschaft des heißen Glasbehälters (30, 64) mit dem Medianwert derselben für eine Vielzahl heißer Glasbehälter (50, 64) vergleicht, die für eine vorbestimmte Zeitspanne unmittelbar vor dem Auswerten des heißen Glasbehälters (30, 64) gelten; und
ein Sichtfeld, auf dem mindestens eines der extrahierten Einzelbilder der heißen Glasbehälter (30, 64) in Echtzeit dargestellt wird;
wobei mindestens eine der Eigenschaften des bzw. der heißen Glasbehälter (30, 64) darstellende diagnostische Informationen entsprechend dem bzw. den extrahierten Bildern auf dem Sichtfeld, die das Vorhandensein bzw. Fehlen einer Abweichung in der mindestens einen Eigenschaft ausweisen, aus den Medianwerten für diese Eigenschaften gleichzeitig auf dem Sichtfeld dargestellt werden.

23. System nach Anspruch 22, bei dem die mindestens eine Abbildungseinrichtung eine kurzwellige Infrarot-Kamera (SWIR) aufweist.

24. System nach Anspruch 22, bei dem die mindestens eine Abbildungseinrichtung eine erste und eine zweite Abbildungsvorrichtung (66, 68) aufweist, die unmittelbar nach der IS-Maschine beiderseits und unter verschiedenen Winkeln bezüglich des Förderers (62) und eines heißen Glasbehälters (64) in dessen Überwachungsposition angeordnet sind.

25. System nach Anspruch 22, bei dem das Bildbearbeitungsmodul (164) aufweist:

ein Modul, das aus den extrahierten Einzelbildern der heißen Glasbehälter (30, 64) den Umriss derselben dimensionshaltig ermittelt; und

ein Modul, das den dimensionshaltigen Umriss der heißen Glasbehälter (30, 64) analysiert, um zu ermitteln, ob diese aneinander haften, umgefallen sind oder fehlen.

26. System nach Anspruch 22, dessen Bildbearbeitungsmodul (164) aufweist:

ein Umrissermittlungsmodul (212, 250), das aus den extrahierten Einzelbildern der heißen Glasbehälter (30, 64) den Umriss jedes heißen Glasbehälters (30, 64) dimensionshaltig ermittelt; und
ein Schiefstandermittlungsmodul (220, 254), das den dimensionshaltigen Umriss des heißen Glasbehälters (30, 64) analysiert, um einen Schiefstand desselben zu ermitteln, wobei der Schiefstand des Glasbehälters (30, 64) als diagnostische Information auf dem Sichtfeld dargestellt wird.

27. System nach Anspruch 22, dessen Bildbearbeitungsmodul (164) aufweist:

ein Modul (234, 258), das aus den extrahierten Einzelbildern der heißen Glasbehälter (30, 64) die vertikale Verteilung des Glass in jedem derselben ermittelt;
wobei die vertikale Glasverteilung im Glasbehälter (30, 64) als diagnostische Information auf dem Sichtfeld dargestellt wird.

28. System nach Anspruch 22, dessen Bildbearbeitungsmodul (164) aufweist:

ein Modul (232, 256), das aus den extrahierten Einzelbildern der heißen Glasbehälter (30, 64) die horizontale Verteilung des Glases in jedem derselben ermittelt;
wobei die horizontale Glasverteilung im Glasbehälter (30, 64) als diagnostische Information auf dem Sichtfeld dargestellt wird.

29. System nach Anspruch 22, dessen Bildbearbeitungsmodul (164) aufweist:

ein Modul (234, 258), das aus den extrahierten Einzelbildern der heißen Glasbehälter (30, 64) die vertikale Verteilung des Glases in jedem derselben ermittelt; und
ein Modul (232, 256), das aus den extrahierten Einzelbildern der heißen Glasbehälter (30, 64) die horizontale Verteilung des Glases in jedem derselben ermittelt,
wobei die vertikale und die horizontale Glasverteilung in den Glasbehältern (30, 64) als diagnostische Information gemeinsam als einziges Bildelement dargestellt werden.

30. System nach Anspruch 22, dessen Bildbearbeitungsmodul (164) aufweist:

eine Datenbank, die mindestens einen programmierbaren Bereich der Bilder der heißen Glasbehälter in einer gewählten Bildhöhe enthält, wobei der programmierbare horizontale Bereich eine programmierbare Höhe von mindestens einer horizontalen Abtastzeile enthält; und
ein Durchmesser-Ermittlungsmodul (236, 260), das aus den extrahierten Einzelbildern der heißen Glasbehälter (30, 64) einen Durchmesser jedes heißen Glasbehälters (30, 64) in jedem programmierbaren horizontalen Bereich ermittelt;
wobei die mindestens entweder den ermittelten Durchmesser oder das Vorhandensein bzw. Fehlen einer Abweichung desselben darstellenden Informationen für den heißen Glasbehälters (30, 64) auf dem Sichtfeld dargestellt werden.

31. System nach Anspruch 30, dessen mindestens eine Abbildungseinrichtung aufweist:

eine erste und eine zweite Abbildungsvorrichtung (66, 68), die unmittelbar nach der IS-Maschine beiderseits des und unter verschiedenen Winkeln zu dem Förderer (62) angeordnet sind, auf dem die heißen Glasbehälter (30, 64) von der IS-Maschine abgeführt werden;
wobei das Durchmesser-Ermittlungsmodul (236, 260) einen Durchmesser jedes heißen Glasbehälters (30, 64) in jedem programmierbaren horizontalen Bereich aus den extrahierten Einzelbildern der heißen Glasbehälter (30, 64) aus dem ersten und zweiten Abbildungsmodul (66, 68) ermittelt; und
wobei, wenn die von der ersten und der zweiten Abbildungsvorrichtung (66, 68) ermittelten Durchmesser um mehr als einen vorbestimmten Betrag für einen heißen Glasbehälter (30, 64) variieren, ein Alarm bzw. eine Warnung ausgelöst oder der heiße Glasbehälter (30, 64) abgewiesen wird.

32. System nach Anspruch 22, dessen Bildbearbeitungsmodul (164) jedem einer Vielzahl von Bereichen der von heißen Glasbehältern (30, 64) abgegeben Strahlung eine Farbe zuweist und aus jedem extrahieren Bild des heißen Glasbehälters (30, 64) ein Farbbild erzeugt, indem es jedem Bildpunkt der extrahierten Einzelbilder der heißen Glasbehälter (30, 64) die seinem Strahlungsbereich zugehörige Farbe zuweist, wobei die aus den extrahierten Einzelbildern der heißen Glasbehälter (30, 64) erzeugten Farbbilder auf dem Sichtfeld dargestellt werden.

33. System nach Anspruch 22, bei dem die IS-Maschine eine bestimmte Anzahl von Abschnitten und in jedem Abschnitt eine bestimmte Anzahl von Formen aufweist,
wobei das Bildbearbeitungsmodul (164) durch Berechnen der Summe der digitalen Werte aller Bildpunkte in allen horizontalen Abtastzeilen im Bild jedes Glasbehälters eine diagnostische Informationsdarstellung der Temperaturen jedes der heißen Glasbehälter (30, 64) ermittelt;
wobei das Bildbearbeitungsmodul (164) für jede Form die in der FIFO-Datenbank gespeicherten Medianwerte der diagnostischen Informationsdarstellung der Temperaturen jedes der heißen Glasbehälter für eine vorbestimmte Zeitspanne ermittelt;
wobei das Bildbearbeitungsmodul (164) für alle Formen von der kältesten zur heißesten die Medianwerte sowie aus den Medianwerten eine Best-fit- bzw. Ausgleichsgerade als Kurve erstellt; und
wobei das Bildbearbeitungsmodul (164) für jede Form die Differenz zwischen der diagnostischen Informationsdarstellung der Temperaturen jedes der heißen Glasbehälter (30, 64) und der Best-fit-Gerade am Ort der gleichen Form ermittelt.

34. System nach Anspruch 22, bei dem die IS-Maschine eine bestimmte Anzahl von Abschnitten und in jedem Abschnitt eine bestimmte Anzahl von Formen aufweist;
wobei das Sichtfeld ein extrahiertes Einzelbild eines in jeder Form jedes Abschnitts ausgebildeten heißen Glasbehälters (30, 64) auf dem Sichtfeld gleichzeitig darstellt und das dargestellte extrahierte Einzelbild eines in jeder Form jedes Abschnitts ausgebildeten heißen Glasbehälters (30, 64) in Echtzeit aktualisiert wird.

35. System nach Anspruch 22, bei dem die IS-Maschine eine bestimmte Anzahl von Abschnitten und in jedem Abschnitt eine bestimmte Anzahl von Formen aufweist, wobei das Sichtfeld die drei extrahierten Einzelbilder der heißen Glasbehälter (30, 64) aus den drei Formen mit den stärksten Abweichungen in der mindestens einen Eigenschaft gleichzeitig darstellt.

36. System nach Anspruch 22, dessen Bildbearbeitungsmodul (164) ein Produktortungsmodul (246) zum Ermitteln des Orts jedes der heißen Glasbehälter (30, 64) auf dem Förderer aufweist, wobei den Ort jedes der heißen Glasbehälter (30, 64) darstellende Informationen an das Sichtfeld gegeben werden.

37. System nach Anspruch 22, dessen Bildbearbeitungsmodul (164) eine FIFO-Datenbank aufweist, in der eine oder mehr der Eigenschaften jedes der heißen Glasbehälter (30, 64) darstellende Informationen für eine vorbestimmten Zeitraum gespeichert sind;
wobei das Bildbearbeitungsmodul (164) den Medianwert der diagnostischen Informationen, die eine oder mehr der Eigenschaften jedes der heißen Glasbehälter (30, 64) für einen vorbestimmten Zeitraum in der FIFO-Datenbank darstellen, ermittelt und die eine oder mehr der Eigenschaften darstellenden diagnostischen Informationen jedes der heißen Glasbehälter (30, 64) mit dem Medianwert vergleicht.

38. System nach Anspruch 37, bei dem die diagnostische Informationen, die einer oder mehr der Eigenschaften jedes der heißen Glasbehälter (30, 64) für einen vorbestimmten Zeitraum in der FIFO-Datenbank entsprechen, sowie deren Medianwert gemeinsam mit der diagnostischen Informationsdarstellung der einen oder mehr Eigenschaften jedes der heißen Glasbehälter (30, 64) als Kastengrafik (Boxplot) auf dem Sichtfeld dargestellt werden.

39. System nach Anspruch 37, bei dem, falls der Vergleich der diagnostischen Information für eine oder mehr der Eigenschaften eines heißen Glasbehälters (30, 64) mit dem Medianwert einen vorbestimmten Prozentwert übersteigt, ein Alarm bzw. eine Warnmeldung ausgelöst oder der heiße Glasbehälter (30, 64) abgewiesen wird.

40. System nach Anspruch 22, bei dem das Sichtfeld eine Betriebsart "Abschnittszusammenfassung" aufweist, in der mindestens drei der Größen "vertikale Glasverteilung", "horizontale Glasverteilung", "Glasverteilung", "Temperatur" und Schiefstand" auf dem Sichtfeld gleichzeitig dargestellt werden.

41. System nach Anspruch 22, das zusätzlich eine IS-Maschinensteuerung (84) aufweist, die mittels der diagnostischen Informationen den Formgebungsprozess in der IS-Maschine selbsttätig steuert.

**42.** System nach Anspruch 22, dessen Sichtfeld eine Benutzerschnittstelle mit berührungsempfindlichem Bildschirm aufweist.

**Revendications**

**1.** Procédé de surveillance et d'analyse des caractéristiques de contenants de verre chaud (30, 64) formés par une machine sectionnelle, le procédé comprenant les étapes:

surveiller, avec au moins un dispositif d'imagerie (42 ; 66, 68), le rayonnement émis par des contenants de verre chaud (30, 64) immédiatement après qu'ils sont formés et avant qu'ils soient refroidis lorsqu'ils sont acheminés depuis la machine sectionnelle sur un transporteur (50, 62) ;
extraire une image individuelle de chacun des contenants de verre chaud (30, 64) à partir du rayonnement surveillé émis par les contenants de verre chaud (30, 64) ;
**caractérisé en ce que** le procédé effectue les étapes :

analyser chaque image individuelle extraite afin d'identifier la présence ou l'absence d'écart dans le procédé de formation de contenants de verre dans chacune d'une pluralité de caractéristiques des contenants de verre chaud (30, 64) en comparant chaque caractéristique du contenant de verre chaud (30, 64) à celle médiane des valeurs pour cette caractéristique dans une pluralité de contenants de verre chaud (30, 64) surveillés pendant une période prédéterminée précédant immédiatement l'évaluation du contenant de verre chaud (30, 64) ;
afficher une ou plusieurs des images individuelles extraites des contenants de verre chaud (30, 64) en temps réel sur un afficheur ; et
afficher simultané sur l'afficheur des informations diagnostiques représentant une ou plusieurs des caractéristiques du ou de chaque contenant de verre chaud (30, 64) correspondant à la ou chaque image individuelle extraite montrée sur l'afficheur et indiquant la présence ou l'absence d'un écart dans l'au moins une caractéristique par rapport à la valeur médiane pour ces caractéristiques.

**2.** Procédé selon la revendication 1, dans lequel le au moins un dispositif d'imagerie comprend :

une caméra infrarouge à courte longueur d'onde (IRCL).

**3.** Procédé selon la revendication 1, dans lequel l'au moins un dispositif d'imagerie comprend :

des premier et second dispositifs d'imagerie (66, 68) positionnés directement après la machine sectionnelle sur des côtés opposés du et à différents angles par rapport au transporteur (62) et au contenant de verre chaud (64) surveillé.

**4.** Procédé selon la revendication 1, dans lequel l'étape d'analyse comprend :

déterminer le contour dimensionnel de chaque contenant de verre chaud (30, 64) à partir des images individuelles extraites du contenant de verre chaud (30, 64) ; et
analyser le contour dimensionnel du contenant de verre chaud (30, 64) afin de déterminer si le contenant de verre chaud (30, 64) est un « article collé », un « article renversé » ou « manquant ».

**5.** Procédé selon la revendication 1, dans lequel l'étape d'analyse comprend :

Déterminer le contour dimensionnel de chaque contenant de verre chaud (30, 64) à partir des images individuelles extraites du contenant de verre chaud (30, 64) ; et
analyser le contour dimensionnel du contenant de verre chaud (30, 64) afin de déterminer toute inclinaison dans le contenant de verre chaud (30, 64) ;
dans lequel l'inclinaison du contenant de verre (30, 64) est affichée sur l'afficheur en tant qu'informations diagnostiques.

**6.** Procédé selon la revendication 1, dans lequel l'étape d'analyse comprend :

déterminer la distribution verticale de verre dans chaque contenant de verre chaud (30, 64) à partir des images individuelles extraites du contenant de verre chaud (30, 64) ;
dans lequel la distribution verticale de verre dans le contenant de verre (30, 64) est affichée sur l'afficheur en tant qu'informations diagnostiques.

7. Procédé selon la revendication 1, dans lequel l'étape d'analyse comprend :

déterminer la distribution horizontale de verre dans chaque contenant de verre chaud (30, 64) à partir des images individuelles extraites du contenant de verre chaud (30, 64) ;
dans lequel la distribution verticale de verre dans le contenant de verre chaud (30, 64) est affichée sur l'afficheur en tant qu'informations diagnostiques.

8. Procédé selon la revendication 1, dans lequel l'étape d'analyse comprend :

déterminer la distribution verticale de verre dans chaque contenant de verre chaud (30, 64) à partir des images individuelles extraites du contenant de verre chaud (30, 64) ;
déterminer la distribution horizontale de verre dans chaque contenant de verre chaud (30, 64) à partir des images individuelles extraites du contenant de verre chaud (30, 64) ;
dans lequel la distribution verticale et la distribution horizontale de verre dans le contenant de verre (30, 64) sont toutes deux affichées sur l'afficheur en tant qu'informations diagnostiques comme un seul élément d'affichage.

9. Procédé selon la revendication 1, dans lequel l'étape d'analyse comprend :

régler au moins une région horizontale programmable sur les images des contenants de verre chaud (30, 64) à une hauteur sélectionnée sur les images de contenants de verre chaud (30, 64), la région horizontale programmable ayant une hauteur programmable d'au moins une ligne de balayage horizontale ;
la détermination d'un diamètre de chaque contenant de verre chaud (30, 64) au niveau de chaque région horizontale programmable à partir des images individuelles extraites du contenant de verre chaud (30, 64) ; et
fournir au moins l'un du diamètre déterminé ou des informations relatives à la présence ou l'absence d'un écart dans le diamètre déterminé pour le contenant de verre chaud (30, 64) à l'afficheur.

10. Procédé selon la revendication 9, dans lequel l'au moins un dispositif d'imagerie comprend :

des premier et second dispositifs d'imagerie (66, 68) positionnés directement après la machine sectionnelle sur des côtés opposés du et à différents angles par rapport au transporteur (62) sur lequel les contenants de verre chaud (64) sont transportés depuis la machine sectionnelle ;
la détermination d'un diamètre de chaque contenant de verre chaud (64) au niveau de chaque région horizontale programmable à partir des images individuelles extraites du contenant de verre chaud (64) à partir de chacun des premier et second dispositifs d'imagerie (66, 68) ; et
si les diamètres déterminés à partir des premier et second dispositifs d'imagerie varient de plus d'une quantité prédéterminée pour un contenant de verre chaud (64), la fourniture d'une alarme ou d' un avertissement ou le rejet du contenant de verre chaud (64).

11. Procédé selon la revendication 1, dans lequel l'étape d'analyse comprend :

attribuer une couleur à chacune d'une pluralité de plages de rayonnement émis par les contenants de verre chaud (30, 64) ;
générer une image colorée à partir de chaque image extraite des contenants de verre chaud (30, 64) en attribuant la couleur associée à la plage de rayonnement pour chaque pixel des images individuelles extraites des contenants de verre chaud (30, 64) ;
dans lequel l'étape d'affichage comprend l'affichage de l'image colorée générée à partir des images individuelles extraites des contenants de verre chaud (30, 64).

12. Procédé selon la revendication 1, dans lequel la machine sectionnelle a un nombre déterminé de sections et un nombre déterminé de moules dans chaque section, dans lequel l'étape d'analyse comprend :

déterminer informations diagnostiques de température représentant les températures de chacun des contenants de verre chaud (30, 64) en calculant la somme des valeurs numériques de tous les pixels sur la totalité d'une pluralité de lignes de balayage horizontales sur l'image de chaque contenant de verre (30, 64) ;

déterminer, pour chaque moule, la valeur médiane des informations diagnostiques de température représentant les températures de chacun des contenants de verre chaud (30, 64) pendant une période prédéterminée stockées dans une base de données premier entré, premier sorti ;

tracer des valeurs médianes par moule du moule le plus froid au moule le plus chaud, et le traçage d'une droite de meilleur ajustement à partir des valeurs médianes ; et

déterminer, pour chaque moule, la différence entre les informations diagnostiques de température représentant les températures de chacun des contenants de verre chaud (30, 64) et la droite de meilleur ajustement à l'emplacement du même moule.

**13.** Procédé selon la revendication 1, dans lequel la machine sectionnelle a un nombre déterminé de sections et un nombre déterminé de moules dans chaque section, dans lequel l'étape d'affichage comprend :

afficher simultané une image individuelle extraite d'un contenant de verre chaud (30, 64) formé dans chacun des moules dans chacune des sections sur l'afficheur, et la mise à jour de l'image individuelle extraite d'un contenant de verre chaud formé dans chacun des moules dans chacune des sections en temps réel.

**14.** Procédé selon la revendication 1, dans lequel la machine sectionnelle a un nombre déterminé de sections et un nombre déterminé de moules dans chaque section, dans lequel l'étape d'affichage comprend :

afficher simultané les trois images individuelles extraites des contenants de verre chaud (30, 64) des trois moules ayant les écarts les plus grands dans l'au moins une caractéristique.

**15.** Procédé selon la revendication 1, dans lequel l'étape d'analyse comprend :

déterminer l'emplacement de chacun des contenants de verre chaud (30, 64) sur le transporteur (50, 62) ; et

dans lequel la position de chacun des contenants de verre chaud (30, 64) est affichée sur l'afficheur.

**16.** Procédé selon la revendication 1, comprenant en outre :

stocker d e s informations diagnostiques représentant une ou plusieurs des caractéristiques de chacun des contenants de verre chaud (30, 64) pendant une période prédéterminée dans une base de données premier entré, premier sorti ;

déterminer la médiane des informations diagnostiques représentant une ou plusieurs des caractéristiques de chacun des contenants de verre chaud (30, 64) pendant une période prédéterminée stockées dans la base de données premier entré, premier sorti ; et

comparer les informations diagnostiques représentant une ou plusieurs des caractéristiques de chacun des contenants de verre chaud (30, 64) à la médiane.

**17.** Procédé selon la revendication 16, dans lequel un diagramme à surfaces des informations diagnostiques représentant une ou plusieurs des caractéristiques de chacun des contenants de verre chaud (30, 64) pendant une période prédéterminée stockées dans la base de données premier entré, premier sorti et sa médiane sont affichées conjointement avec les informations diagnostiques représentant les une ou plusieurs des caractéristiques de chacun des contenants de verre chaud (30, 64) sur l'afficheur.

**18.** Procédé selon la revendication 16, dans lequel si la comparaison des informations diagnostiques représentant une ou plusieurs des caractéristiques d'un contenant de verre chaud (30, 64) avec la médiane dépasse un pourcentage prédéterminé, une alarme ou un avertissement est prévu ou le contenant de verre chaud (30, 64) est rejeté.

**19.** Procédé selon la revendication 1, dans lequel les informations diagnostiques simultanément affichées sur l'afficheur dans un mode de résumé de section comprennent :

au moins trois éléments parmi la distribution de verre verticale, la distribution de verre horizontale, la distribution de verre, la température et l'inclinaison.

**20.** Procédé selon la revendication 1, comprenant en outre :

l'utilisation des informations diagnostiques pour commander automatiquement le procédé de formation dans la machine sectionnelle

21. Procédé selon la revendication 1, dans lequel l'afficheur comprend :

une interface utilisateur à écran tactile.

22. Système de surveillance et d'analyse de caractéristiques de contenants de verre chaud (30, 64) formés par une machine sectionnelle, le système comprenant :

au moins un dispositif d'imagerie (42 ; 66, 68), qui surveille le rayonnement émis par des contenants de verre chaud (30, 64) immédiatement après qu'ils sont formés et avant qu'ils soient refroidis lorsqu'ils sont acheminés depuis la machine sectionnelle sur un transporteur (50, 62) ;
un module d'extraction d'image (180, 242) qui extrait une image individuelle de chacun des contenants de verre chaud (30, 64) à partir du rayonnement surveillé émis par les contenants de verre chaud (30, 64) ;
**caractérisé par** :

un module de traitement d'image (164) qui analyse chaque image individuelle extraite afin d'identifier la présence ou l'absence d'écart dans le procédé de formation de contenants de verre (30, 64) dans chacune d'une pluralité de caractéristiques des contenants de verre chaud (30, 64) en comparant chaque caractéristique du contenant de verre chaud (30, 64) à celle médiane des valeurs pour cette caractéristique dans une pluralité de contenants de verre chaud (30, 64) surveillés pendant une période prédéterminée précédant immédiatement l'évaluation du contenant de verre chaud (30, 64) ; et
un afficheur sur lequel au moins l'une des images individuelles extraites des contenants de verre chaud (30, 64) est affichée en temps réel ;
dans lequel les informations diagnostiques représentant au moins l'une des caractéristiques du ou de chaque contenant de verre chaud (30, 64) correspondant à la ou chaque image individuelle extraite montrée sur l'afficheur et indiquant la présence ou l'absence d'un écart dans l'au moins une caractéristique par rapport aux valeurs médianes de ces caractéristiques sont simultanément affichées sur l'afficheur.

23. Système selon la revendication 22, dans lequel l'au moins un dispositif d'imagerie comprend :

une caméra infrarouge à courte longueur d'onde (IRCL).

24. Système selon la revendication 22, dans lequel l'au moins un dispositif d'imagerie comprend :

des premier et second dispositifs d'imagerie (66, 68) positionnés directement après la machine sectionnelle sur des côtés opposés du et à différents angles par rapport au transporteur (62) et au contenant de verre chaud (64) lorsqu'il est positionné pour être surveillé.

25. Système selon la revendication 22, dans lequel le module de traitement d'image (164) comprend :

un module de détermination de contour qui détermine le contour dimensionnel de chaque contenant de verre chaud (30, 64) à partir des images individuelles extraites du contenant de verre chaud (30, 64) ; et
un module qui analyse le contour dimensionnel du contenant de verre chaud (30, 64) afin de déterminer si le contenant de verre chaud (30, 64) est un « article collé », un « article renversé » ou « manquant ».

26. Système selon la revendication 22, dans lequel le module de traitement d'image (164) comprend :

un module de détermination de contour (212, 250) qui détermine le contour dimensionnel de chaque contenant de verre chaud (30, 64) à partir des images individuelles extraites du contenant de verre chaud (30, 64) ; et
un module de détermination d'inclinaison (220, 254) qui analyse le contour dimensionnel du contenant de verre chaud (30, 64) afin de déterminer toute inclinaison dans le contenant de verre chaud (30, 64) ;
dans lequel l'inclinaison du contenant de verre (30, 64) est affichée sur l'afficheur en tant qu'informations diagnostiques.

27. Système selon la revendication 22, dans lequel le module de traitement d'image (164) comprend :

un module de détermination de distribution verticale (234, 258) qui détermine la distribution verticale de verre dans chaque contenant de verre chaud (30, 64) à partir des images individuelles extraites du contenant de verre chaud (30, 64) ;

dans lequel la distribution verticale de verre dans le contenant de verre (30, 64) est affichée sur l'afficheur en tant qu'informations diagnostiques.

**28.** Système selon la revendication 22, dans lequel le module de traitement d'image (164) comprend :

un module de détermination de distribution horizontale (232, 256) qui détermine la distribution horizontale de verre dans chaque contenant de verre chaud (30, 64) à partir des images individuelles extraites du contenant de verre chaud (30, 64) ;

dans lequel la distribution horizontale de verre dans le contenant de verre (30, 64) est affichée sur l'afficheur en tant qu'informations diagnostiques.

**29.** Système selon la revendication 22, dans lequel le module de traitement d'image (164) comprend :

un module de détermination de distribution verticale (234, 258) qui détermine la distribution verticale de verre dans chaque contenant de verre chaud (30, 64) à partir des images individuelles extraites du contenant de verre chaud (30, 64) ;

un module de détermination de distribution horizontale (232, 256) qui détermine la distribution horizontale de verre dans chaque contenant de verre chaud (30, 64) à partir des images individuelles extraites du contenant de verre chaud (30, 64) ;

dans lequel la distribution verticale et la distribution horizontale de verre dans le contenant de verre (30, 64) sont toutes deux affichées sur l'afficheur en tant qu'informations diagnostiques comme un seul élément d'affichage.

**30.** Système selon la revendication 22, dans lequel le module de traitement d'image (164) comprend :

une base de données contenant au moins une région horizontale programmable sur les images des contenants de verre chaud à une hauteur sélectionnée sur les images des contenants de verre chaud, la région horizontale programmable ayant une hauteur programmable d'au moins une ligne de balayage horizontale ; et

un module de détermination de diamètre (234, 260) qui détermine un diamètre de chaque contenant de verre chaud (30, 64) au niveau de chaque région horizontale programmable à partir des images individuelles extraites du contenant de verre chaud (30, 64) ;

dans lequel les informations diagnostiques représentant au moins l'un du diamètre déterminé ou des informations relatives à la présence ou l'absence d'un écart dans le diamètre déterminé pour le contenant de verre chaud (30, 64) sont fournies à l'afficheur.

**31.** Système selon la revendication 30, dans lequel l'au moins un dispositif d'imagerie comprend :

des premier et second dispositifs d'imagerie (66, 68) positionnés directement après la machine sectionnelle sur des côtés opposés du et à différents angles par rapport au transporteur (62) sur lequel les contenants de verre chaud (30, 64) sont transportés depuis la machine sectionnelle ;

dans lequel le module de détermination de diamètre (236, 260) détermine un diamètre de chaque contenant de verre chaud (30, 64) au niveau de chaque région horizontale programmable à partir des images individuelles extraites du contenant de verre chaud (30, 64) à partir de chacun des premier et second dispositifs d'imagerie (66, 68) ; et

dans lequel si les diamètres déterminés à partir des premier et second dispositifs d'imagerie (66, 68) varient de plus d'une quantité prédéterminée pour un contenant de verre chaud (30, 64), une alarme ou un avertissement est prévu ou le contenant de verre chaud (30, 64) est rejeté.

**32.** Système selon la revendication 22, dans lequel le module de traitement d'image (164) attribue une couleur à chacune d'une pluralité de plages de rayonnement émis par des contenants de verre chaud (30, 64), et dans lequel le module de traitement d'image (164) génère une image colorée à partir de chaque image extraite des contenants de verre chaud (30, 64) en attribuant la couleur associée à la plage de rayonnement pour chaque pixel des images individuelles extraites des contenants de verre chaud (30, 64) ; et

dans lequel les images colorées générées à partir des images extraites des contenants de verre chaud (30, 64) sont affichées sur l'afficheur.

**33.** Système selon la revendication 22, dans lequel la machine sectionnelle a un nombre déterminé de sections et un nombre déterminé de moules dans chaque section, dans lequel le module de traitement d'image (164) détermine la représentation d'informations diagnostiques de température des températures de chacun des contenants de verre chaud (30, 64) en calculant la somme des valeurs numériques de tous les pixels sur la totalité des lignes de balayage horizontales sur l'image de chaque contenant de verre ;

dans lequel le module de traitement d'image (164) détermine, pour chaque moule, la valeur médiane des informations diagnostiques de température représentant les températures de chacun des contenants de verre chaud pendant une période prédéterminée stockées dans la base de données premier entré, premier sorti ;

dans lequel le module de traitement d'image (164) trace les valeurs médianes par moule du moule le plus froid au moule le plus chaud, et trace une droite de meilleur ajustement à partir des valeurs médianes ; et

dans lequel le module de traitement d'image (164) détermine, pour chaque moule, la différence entre les informations diagnostiques de température représentant les températures de chacun des contenants de verre chaud (30, 64) et la droite de meilleur ajustement à l'emplacement du même moule.

**34.** Système selon la revendication 22, dans lequel la machine sectionnelle a un nombre déterminé de sections et un nombre déterminé de moules dans chaque section, dans lequel l'afficheur affiche simultanément une image individuelle extraite d'un contenant de verre chaud (30, 64) formé dans chacun des moules dans chacune des sections sur l'afficheur, et l'image individuelle extraite affichée d'un contenant de verre chaud (30, 64) formé dans chacun des moules dans chacune des sections est mise à jour en temps réel.

**35.** Système selon la revendication 22, dans lequel la machine sectionnelle a un nombre déterminé de sections et un nombre déterminé de moules dans chaque section, dans lequel l'afficheur affiche simultanément les trois images individuelles extraites des contenants de verre chaud (30, 64) des trois moules ayant les écarts les plus grands dans l'au moins une caractéristique.

**36.** Système selon la revendication 22, dans lequel le module de traitement d'image (164) comprend :

un module de localisation de produit (246) permettant de déterminer l'emplacement de chacun des contenants de verre chaud (30, 64) sur le transporteur ; et

dans lequel des informations représentant la position de chacun des contenants de verre chaud (30, 64) sont fournies à l'afficheur.

**37.** Système selon la revendication 22, dans lequel le module de traitement d'image (164) comprend :

une base de données premier entré, premier sorti qui stocke des informations diagnostiques représentant une ou plusieurs des caractéristiques de chacun des contenants de verre chaud (30, 64) pendant une période prédéterminée ;

dans lequel le module de traitement d'image (164) détermine la médiane des informations diagnostiques représentant une ou plusieurs des caractéristiques de chacun des contenants de verre chaud (30, 64) pendant une période prédéterminée stockées dans la base de données premier entré, premier sorti ; et compare les informations diagnostiques représentant une ou plusieurs des caractéristiques de chacun des contenants de verre chaud (30, 64) à la médiane.

**38.** Système selon la revendication 37, dans lequel un diagramme à surfaces des informations diagnostiques représentant une ou plusieurs des caractéristiques de chacun des contenants de verre chaud (30, 64) pendant une période prédéterminée stockées dans la base de données premier entré, premier sorti et sa médiane sont affichés conjointement avec les informations diagnostiques représentant les une ou plusieurs des caractéristiques de chacun des contenants de verre chaud (30, 64) sur l'afficheur.

**39.** Système selon la revendication 37, dans lequel si la comparaison des informations diagnostiques représentant une ou plusieurs des caractéristiques d'un contenant de verre chaud (30, 64) avec la médiane dépasse un pourcentage prédéterminé, une alarme ou un avertissement est prévu ou le contenant de verre chaud (30, 64) est rejeté.

**40.** Système selon la revendication 22, dans lequel l'afficheur comprend un mode de résumé de section dans lequel au moins trois éléments parmi la distribution de verre verticale, la distribution de verre horizontale, la distribution de verre, la température et l'inclinaison sont simultanément affichés sur l'afficheur.

**41.** Système selon la revendication 22, comprenant en outre :

une unité de commande de machine sectionnelle (84) qui utilise les informations diagnostiques pour commander automatiquement le procédé de formation dans la machine sectionnelle

**42.** Système selon la revendication 22, dans lequel l'afficheur comprend :

une interface utilisateur à écran tactile.

FIG. 1

Container Image

FIG. 2

Line Radiation Measurement

FIG. 3

Measurement Ratio

FIG. 4

Reference

FIG. 5

FIG. 6

FIG. 7

FIG. 8

FIG. 9

FIG. 10

FIG. 11

EP 2 333 502 B1

# FIG. 14

# FIG. 13

184

186

182

# FIG. 12

IMAGE PROCESSING MODULE 1

LINE SCAN CAMERA1 INPUT

IMAGE OUTPUT

CAMERA2 REPORT DATA

CAMERA3 REPORT DATA

164

COMBINE MODULE

COMBINED REPORT DATA

REJECT FLAG

194

180 — EXTRACT INTO INDIVIDUAL IMAGES

188 — STUCK WARE AND DOWN WARE DETECTION

196 — PRODUCT LOCATION

212 — DETERMINE OUTLINE

216 — DETERMINE MIDDLE

220 — DETERMINE LEAN

232 — DETERMINE HORIZONTAL DISTRIBUTION

234 — DETERMINE VERTICAL DISTRIBUTION

236 — DETERMINE DIAMETER

186

FIG. 15

FIG. 18

FIG. 19

FIG. 20

FIG. 21

190

214

186

224

186

218

222

226

228

230

FIG. 16

FIG. 17

192

64

64

64

64

64

64

64

62   210     208     206     204     202     200     198

EP 2 333 502 B1

## FIG. 22

```
┌──────────┐  ┌──────────┐  ┌──────────┐   ┌──────────┐   ┌──────────┐   ┌──────┐  ┌──────┐  ┌──────┐  ┌──────────┐
│  240     │  │  244     │  │  246     │   │  250     │   │  252     │   │ 254  │  │ 256  │  │ 258  │  │  260     │
│ CAMERA 1 │  │STUCK WARE│  │          │   │CONTAINER │   │CONTAINER │   │      │  │HORIZ.│  │VERT. │  │          │
│  IMAGE   │  │& DOWN WARE│ │ PRODUCT  │   │OUTLINE   │   │MIDDLE -  │   │ LEAN │  │DISTRI│  │DISTRI│  │ DIAMETER │
│          │  │ BASED ON │  │ LOCATION │   │(EDGE     │   │CENTER LINE│  │      │  │-BUTION│ │-BUTION│ │          │
│          │  │  WIDTH   │  │          │   │DETECTION)│   │BEST FIT LINE││      │  │      │  │      │  │          │
└──────────┘  └──────────┘  └──────────┘   └──────────┘   └──────────┘   └──────┘  └──────┘  └──────┘  └──────────┘
```

EXTRACT BOTTLE IMAGE FROM FILM STRIP — 242

FORMATTED IMAGE DATA — 248

```
                ┌──────────────┐      ┌──────────┐
                │  262         │      │COMBINED  │
                │ REPORTS      │ ───► │REPORT    │
                │ FROM OTHER   │      │DATA      │
                │ CAMERA       │      │          │
                │ MODULE(S)    │      └──────────┘
                └──────────────┘          264
```

## FIG. 24

```
┌──────────┐
│  292     │
│ CAMERA 2 │
│  IMAGE   │
│ AND DATA │
└──────────┘

┌──────────┐  ┌──────────┐  ┌──────────┐  ┌──────┐  ┌──────────┐  ┌──────────────┐  ┌──────────┐  ┌──────────┐  ┌──────────┐
│  290     │  │  296     │  │  298     │  │ 302  │  │  304     │  │  306         │  │  308     │  │  310     │  │  312     │
│ CAMERA 1 │  │ COMBINED │  │ CAMERA   │  │ X, Y │  │ TOTAL LEAN│ │ HORIZONTAL   │  │ VERTICAL │  │ DIAMETER │  │ TEMPER-  │
│  IMAGE   │─►│ REPORT   │◄►│ POSITION │  │POSITION│ │ (BOTH    │  │ DISTRIBUTION │  │DISTRIBUTION│ │(CAMERA 1 │  │ ATURE    │
│ AND DATA │  │ DATA     │  │ ANGLE    │  │      │  │ CAMERAS) │  │ BOTH CAMERAS │  │(CAMERA 1 │  │  ONLY)   │  │(CAMERA 1 │
│          │  │          │  │ AND TIME │  │      │  │          │  │ CENTER OF    │  │  ONLY)   │  │          │  │  ONLY)   │
│          │  │          │  │ POSITION │  │      │  │          │  │ INTENSITY    │  │          │  │          │  │          │
│          │  │          │  │          │  │      │  │          │  │ CALCULATION  │  │          │  │          │  │          │
└──────────┘  └──────────┘  └──────────┘  └──────┘  └──────────┘  └──────────────┘  └──────────┘  └──────────┘  └──────────┘

┌──────────┐
│ CAMERA 3 │
│  IMAGE   │
│ AND DATA │
│  294     │
└──────────┘
```

COMBINED OBJECT DATA — 300

EP 2 333 502 B1

42

# FIG. 23

FIG. 25

EP 2 333 502 B1

FIG. 26

Vertical distribution

Horizontal distribution

Combined distribution

**FIG. 27A**

**FIG. 27B**

**FIG. 27C**

FIG. 28

FIG. 29

FIG. 30

FIG. 31

FIG. 32

FIG. 33

EP 2 333 502 B1

FIG. 34

53

FIG. 35

FIG. 36

FIG. 37

FIG. 38

FIG. 39

FIG. 40

## REFERENCES CITED IN THE DESCRIPTION

**Patent documents cited in the description**

- US 5583337 A, Chan
- EP 643297 A, Troost
- US 6188079 B, Juvinall
- US 20060096319 A, Dalstra
- EP 09075545 A
- EP 2336740 A